# EUROPEAN PATENT APPLICATION

(11) **EP 1 468 694 A1**
(43) Date of publication of application: **20.10.2004**
(21) Application number: 02790920.9
(22) Date of filing: 27.12.2002
(51) Int. Cl.: A61K 38/00, A61K 45/00, A61P 3/04, A61P 3/06, A61P 3/10

(54) **REMEDIES FOR ANOREXIA OR LIFESTYLE-RELATED DISEASES AND METHOD OF SCREENING THE SAME**

(30) Priority: 27.12.2001 JP 2001397523
(71) Applicant: Sumitomo Pharmaceuticals Company, Limited, Osaka 541-8510 (JP)
(72) Inventor: SUGARU, Eiji, Osaka 554-0013 (JP); YAMANAKA, Mitsugu, Toyonaka-shi, Osaka 561-0802 (JP); ICHIHARA, Junji, Ibaraki-shi, Osaka 567-0841 (JP); TAIJI, Mutsuo, Takatsuki-shi, Osaka 569-1044 (JP)
(74) Representative: VOSSIUS & PARTNER
(86) International application number: PCT/JP2002/013757
(87) International publication number: WO 2003/055507

(57) **Abstract**

The present invention provides a novel therapeutic agent for a lifestyle-related disease or a cibophobia, which is superior in controlling food intake, and a screening method therefor. Specifically, a therapeutic agent for cibophobia comprising, as an active ingredient, a substance that suppresses expression or function of GPRC5D, GPCR expressed in hypothalamus, a therapeutic agent for a lifestyle-related disease comprising, as an active ingredient, a substance that enhances expression or function of the receptor, a screening system consisting of a series of coexpression systems of GPRC5D and various G proteins, and a screening method for a substance having a therapeutic activity against cibophobia or a lifestyle-related disease, which includes use of the screening system, are provided.

## Description

### Technical Field

The present invention relates to a therapeutic agent for cibophobia, which comprises, as an active ingredient, a substance that suppresses expression or function of orphan GPCR expressed in hypothalamus. Specifically, the present invention relates to a therapeutic agent for cibophobia, which comprises, as an active ingredient, antisense nucleic acid of GPCR mRNA, an expression vector containing the nucleic acid or a host cell transfected with the expression vector, or a therapeutic agent for cibophobia, which comprises, as an active ingredient, a substance having an antagonist activity to GPCR. Furthermore, the present invention relates to a coexpression system of a GPCR and a G protein and a screening method for a therapeutically active compound for cibophobia using the same. The present invention moreover relates to a therapeutic agent for a lifestyle-related disease, which comprises, as an active ingredient, a substance that enhances the expression or function of GPCR. In detail, the present invention relates to a therapeutic agent for a lifestyle-related disease, which comprises, as an active ingredient, a substance having an agonist activity to GPCR. Furthermore, the present invention relates to a coexpression system of a GPCR and a G protein and a screening method for a therapeutically active compound for a lifestyle-related disease using the same.

### Background Art

Due to westernization of the eating habits, increase of social stress and the like in recent years, the number of patients with obesity and accompanying lifestyle-related diseases, particularly type II diabetes, has been increasing dramatically. For the therapy of these cases, exercise therapy and diet therapy are performed first. When the weight control is insufficient even by these treatments, drug therapy is performed. In doing so, a therapeutic agent which is superior in controlling food intake, body weight and blood glucose, and which is safe has been desired.

On the other hand, stress society of the present age combined with epidemic of an easy diet has brought a rapid increase of psychogenic eating disorder, such as cibophobia, in adolescent women. While it is indispensable to solve psychological problems to treat these diseases fundamentally, drug therapy may be performed to forcibly control feeding behavior as a supportive therapy. A therapeutic agent in this case is also requested to be able to promote eating while controlling body weight and glucose level.

Eating is mainly controlled by the central nervous system, and many nervous systems ruling over instinctive behaviors of human, such as appetite and the like, are located particularly in hypothalamus. In fact, when hypothalamus ventromedial nucleus of rat is damaged, it causes overeating and obesity, whereas when hypothalamus lateral nucleus is damaged, feeding behavior is not taken. In addition, localization of receptors of leptin and neuropeptides (e.g., neuropeptide Y (NPY)), which are involved in eating control, has been shown heretofore, which makes it clear that hypothalamus is an important organ for feeding behavior.

It has become clear that receptors of physiologically active substances in the central nervous system including hypothalamus, particularly G protein-coupled receptor (GPCR), correlates with feeding behavior. For example, it is known that knockout (KO) mouse with serotonin 5-THT₂C receptor suffers from chronic overeating. In addition, melanocortin 4 receptor antagonist increases food intake and, on the contrary, NPY Y5 receptor antagonist suppresses food intake.

Thus, stimulation of the nervous system in hypothalamus is considered to influence the feeding behavior, and substitute operation of signal transduction through GPCR, which is expressed in hypothalamus, by the use of a small compound meets the above-mentioned object of controlling food intake, body weight, glucose level and the like. However, a drug having such an action mechanism has not been marketed at present, and development of such a pharmaceutical agent has been highly desired.

It is therefore an object of the present invention to provide a means for regulating feeding behavior by allowing an external factor, particularly a factor that suppresses or promotes expression or function of GPCR involved in the feeding behavior, to function, thereby treating a lifestyle-related disease mainly caused by overeating or obesity, or cibophobia. It is another object of the present invention to provide a compound having a controlling effect on eating disorder such as overeating and apocleisis, obesity or the like, and a screening method for such a compound.

### Disclosure of the Invention

To achieve the above-mentioned objects, the present inventors first examined a gene encoding a receptor expressed in hypothalamus, and, as a result, found a certain orphan GPCR (hereinafter to be referred to as GPRC5D) gene is expressed in hypothalamus of obese model mouse. Next, the present inventors administered an antisense oligo DNA of mRNA encoding this receptor to obese model mouse to inhibit its expression. As a result, food intake was in fact increased and blood glucose level was elevated. Therefrom it has been clarified that GPRC5D is a receptor involved in the signal transduction which negatively regulates the feeding behavior, and therefore, a substance inhibiting expression or function of this receptor shows a therapeutic effect on eating disorders such as cibophobia. In contrast, a substance enhancing expression or function of this receptor should show a therapeutic effect on lifestyle-related diseases including type II diabetes caused by overeating, obesity and the like. Thus, the present inventors constructed a series of coexpression systems of GPRC5D and various G proteins, and developed a method for screening for a compound having a therapeutic activity against cibophobia or lifestyle-related diseases by searching for an agonist or an antagonist to GPRC5D using the system, which resulted in the completion of the present invention.

Accordingly, the present invention provides a therapeutic agent for cibophobia, which comprises, as an active ingredient, a substance that suppresses expression or function of GPRC5D, a GPCR expressed in hypothalamus. As a substance capable of specifically suppressing the expression of GPRC5D, an antisense nucleic acid of mRNA encoding GPRC5D can be preferably mentioned. In this case, the antisense nucleic acid can be provided not only as it is but in the form of an expression vector encoding said nucleic acid or a host cell into which said expression vector has been introduced. In addition, as a substance capable of specifically suppressing the function of GPRC5D, an antagonist to said receptor can be mentioned.

The present invention further provides a therapeutic agent for lifestyle-related diseases, which comprises, as an active ingredient, a substance enhancing expression or function of GPRC5D. As a substance capable of specifically enhancing the function of GPRC5D, a physiological ligand and an agonist to this receptor can be mentioned.

Therefore, another aspect of the present invention provides a screening method for a substance having a therapeutic activity against cibophobia or lifestyle-related diseases, which comprises screening for an antagonist or an agonist to GPRC5D. This method comprises comparing a GDP/GTP exchange reaction of a G protein or the activity of an effector the G protein acts upon, in the presence and absence of the test substance, in a series of receptor - G protein coexpression systems obtained by constructing a constitution unit for a receptor-binding region of each family of Gα, wherein one constitution unit comprises a system comprising, as essential elements, at least a lipid bilayer membrane comprising GPRC5D or an equivalent thereof, and a polypeptide comprising at least a receptor-binding region of a G protein α subunit (hereinafter to be also referred to as Gα) belonging to a certain family and a guanine nucleotide-binding region of any G protein α subunit.

Accordingly, the present invention also provides a screening system for a substance having a therapeutic activity against cibophobia or a lifestyle-related disease, which comprises the above-mentioned series of receptor-G protein coexpression systems.

The present invention moreover provides a therapeutic agent for cibophobia or a therapeutic agent for a lifestyle-related disease, comprising, as an active ingredient, a substance having a therapeutic activity against cibophobia or a lifestyle-related disease, which is obtained by the above-mentioned screening system or screening method.

Further characteristics and advantages of the present invention will become clear from the disclosure of "Best Mode for Embodying the Invention" below.

### Brief Description of the Drawings

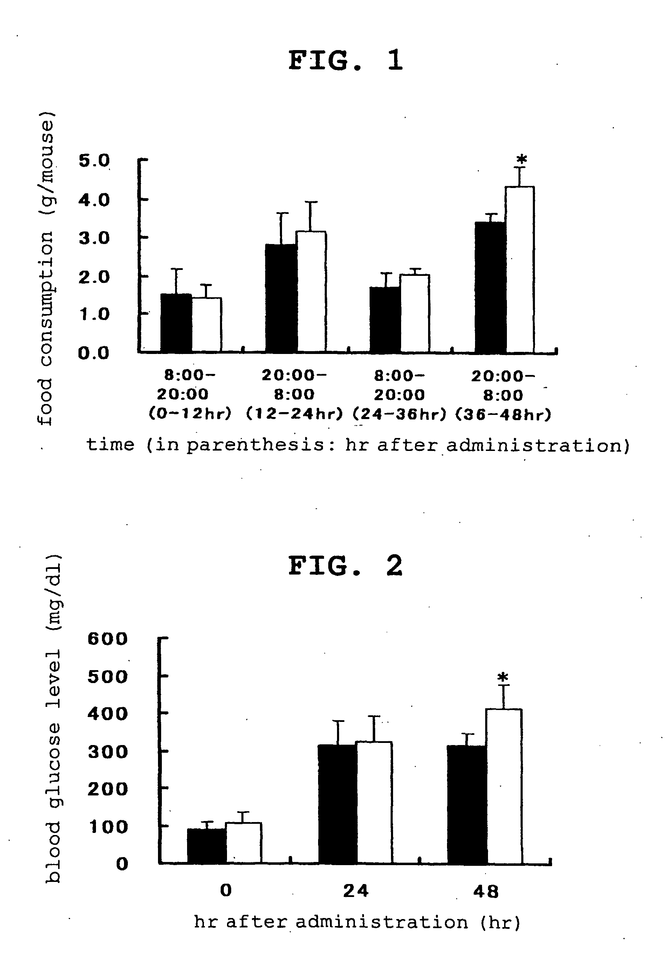

Fig. 1 shows the effect of administration of antisense DNA of GPRC5D on food intake of obese mouse (immediately after administration - 48 hours later), wherein black columns show food consumption of control DNA administration groups, white columns show that of antisense DNA administration groups (average ± standard deviation, control DNA administration groups n=4, antisense DNA administration groups n=4), and * in the Figure indicates the presence of a significant difference between the both groups (p<0.05, Student's t-test).
Fig. 2 shows the effect of administration of antisense DNA of GPRC5D on blood glucose level of obese mouse (immediately after administration - 48 hours later), wherein black columns show blood glucose level of control DNA administration groups, white columns show that of antisense DNA administration groups (average ± standard deviation, control DNA administration groups n=4, antisense DNA administration groups n=4), and * in the Figure indicates the presence of a significant difference between the both groups (p<0.05, Student's t-test).

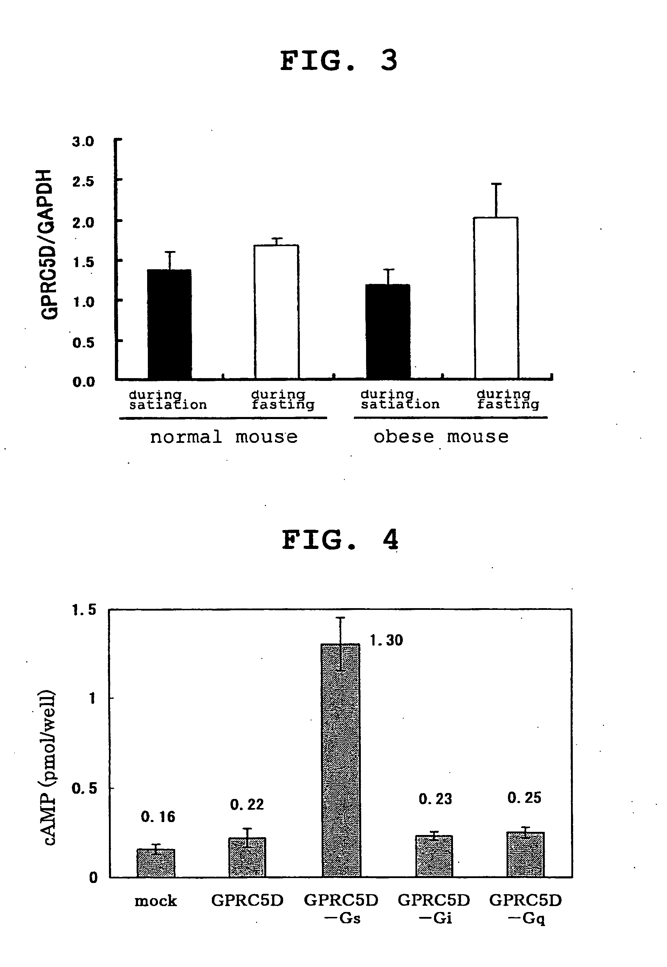

Fig. 3 shows variation in expression of GPRC5D gene in normal mouse and obese mouse in satiation state and fasting state, wherein black columns show ratio of GPRC5D/GAPDH in the satiation state, white columns show that in the fasting state (average ± standard deviation, n=3 in each group).
Fig. 4 shows cAMP concentrations in extract from CHO-K1 cell in which GPRC5D was transiently expressed alone or on fusing with various Gα proteins, wherein mock shows CHO-K1 cells transfected with pcDNA3.1(+), GPRC5D shows CHO-K1 cells transfected with pcC5D, GPRC5D-Gs shows CHO-K1 cells transfected with pcC5D/His/GαS2, GPRC5D-Gi shows CHO-K1 cells transfected with pcC5D/His/Gαi2 and GPRC5D-Gq shows CHO-K1 cells transfected with pcC5D/His/Gα16.

### Best Mode for Embodying the Invention

The present invention provides a therapeutic agent for cibophobia, which comprises as an active ingredient a substance that suppresses the expression or function of GPRC5D, a GPCR expressed in the hypothalamus.

"GPRC5D" is one of human GPCR proteins consisting of the amino acid sequence shown by SEQ ID NO:2, which has been newly discovered by homology search on EST database using a set of human GPCR amino acid sequence categorized into the 5th group of metabotrophic glutamate receptor-like family (family C) (Brauner-Osborne et al., *Biochim*. *Biophys*. *Acta*, *1518*(3): 237-48(2001); registered in GeneBank under accession numbers: AF209923, XM006896 and NM018654). However, physiological functions, physiological ligands and coupling G protein (α subunit) subtypes, etc. of GPRC5D have remained unclear. The present inventors have independently found that this receptor gene is expressed in hypothalamus of the obese model mouse and conducted further investigations based on this finding. As a result, as described above, the present inventors have identified this protein as a membrane receptor involved in stimulation of the feeding center.

It is known that GPCR homologs corresponding to human GPRC5D exist in other mammals [e.g., homolog mGprc5d (SEQ ID NO:4) which has about 82% identity with human GPRC5D at the amino acid level was discovered in mouse (Brauner-Osborne et al., ibid. registered in GenBank under accession number: AF218809). Hereinafter a simple reference to "GPRC5D" is to be understood as collectively referring to human GPRC5D and its mouse homolog.]. Accordingly, the therapeutic agent for cibophobia of the present invention is intended for use to eating disorders not only in humans but also in other mammals. Since social environment of the present age is a stressful environment for animals like livestocks and pets, the present invention is useful for the application to the field of veterinary medicine as well.

The therapeutic agent for cibophobia of the present invention comprises, as an active ingredient, a substance that suppresses the expression or function of GPRC5D. The term "expression", as used herein, refers to a state wherein a receptor protein is produced and functionally located on the cell membrane. Accordingly, the "substance that suppresses the expression" may act at any stage, such as at the gene transcription level, post-transcription regulation level, translation-into-protein level, post-translational modification level, membrane transport level and protein folding level. On the other hand, the "substance that suppresses the function" refers to a substance that acts on a receptor once functionally located on the cell membrane, and that does not cause a shift of the equilibration between the active and inactive forms at least toward the active side.

Examples of substances that suppress the expression of GPRC5D include transcriptional suppressors, RNA polymerase inhibitors, RNA-decomposing enzymes, protein synthesis inhibitors, proteases, and protein denaturants; to minimize the adverse effects on other genes and proteins that are expressed in cells, it is important that the substance should be capable of specifically acting on the target molecule. Accordingly, a preferred embodiment of a substance that suppresses the expression of GPRC5D is an antisense nucleic acid of the GPRC5D mRNA (consisting of the base sequence shown by SEQ ID NO:1 (indicates only ORF) or SEQ ID NO:3) or its initial transcription product. The term "antisense nucleic acid" refers to a nucleic acid that consists of a base sequence capable of hybridizing to target mRNA (initial transcription product) under the physiological conditions of cells that express the target mRNA (initial transcription product), and that is capable of inhibiting the translation into the polypeptide encoded by the target mRNA (initial transcription product) under the hybridized state. The kind of antisense nucleic acid may be DNA or RNA and may be a DNA/RNA chimera. Additionally, because natural form of antisense nucleic acids have their phosphate di-ester linkage decomposed easily by the nuclease present in cells, the antisense nucleic acid of the present invention can also be synthesized using modified nucleotides such as of the thiophosphate type (phosphate bond P=O replaced with P=S) or 2'-O-methyl type, which types are stable to the nuclease. Other important requirements for designing an antisense nucleic acid include increasing the water solubility and cell membrane permeability; these goals can also be achieved by improving the dosage form such as through the use of liposome or microspheres.

The length of the antisense nucleic acid of the present invention is not particularly limited, as long as the antisense nucleic acid is capable of specifically hybridizing to the GPRC5D mRNA or its initial transcription product, and the antisense nucleic acid may be a sequence comprising a sequence of about 15 bases at the shortest in length or complementary to the entire sequence of the mRNA (initial transcription product) at the longest. From the viewpoint of ease of synthesis, antigenicity concern, and other aspects, there may be mentioned, for example, oligonucleotides consisting of preferably about 15 to about 30 bases. When the antisense nucleic acid is an about 25mer oligo DNA, the base sequence capable of hybridizing to the GPRC5D mRNA under physiological conditions may be any one, as long as it possesses about 80% identity or more, depending on the base composition of the target sequence.

The target sequence for the antisense nucleic acid of the present invention is not particularly limited, as long as it is a sequence such that the translation into GPRC5D protein or a functional fragment thereof is inhibited as a result of hybridization with the antisense nucleic acid, and may be the entire sequence or a partial sequence of the GPRC5D mRNA or may be the intron portion of the initial transcription product. However, when using an oligonucleotide as the antisense nucleic acid, it is desirable that the target sequence should be located between the 5'-terminus of the GPRC5D mRNA and the C-terminus of the coding region (the region shown by base numbers 1-1035 in the base sequence shown by SEQ ID NO:1 or base numbers 1-1047 in the base sequence shown by SEQ ID NO:3). Preferably, the target sequence is located on region between the 5'-terminus and the N-terminus side of the coding region, with greatest preference given to a base sequence in the vicinity of the initiation codon (base numbers 148-150 in the base sequence shown by SEQ ID NO:3). Additionally, it is preferable that the target sequence should be selected such that an antisense nucleic acid complementary thereto does not form a secondary structure such as a hairpin structure.

Furthermore, the antisense nucleic acid of the present invention may be capable of not only hybridizing to the GPRC5D mRNA or its initial transcription product to inhibit the translation, but also binding to the GPRC5D gene, a double-stranded DNA, to form a triple-strand (triplex) to inhibit the transcription into mRNA.

Another preferred embodiment of a substance that suppresses GPRC5D expression is a ribozyme capable of specifically cleaving within the coding region of the GPRC5D mRNA or its initial transcription product (the base sequence shown by base numbers 1-1035 in the base sequence shown by SEQ ID NO:1 or base numbers 148-1047 in the base sequence shown by SEQ ID NO:3) (including the intron portion in case of the initial transcription product). The term "ribozyme" refers to an RNA having an enzyme activity to cleave nucleic acid. Since it has recently been shown that oligo DNA having the base sequence of the enzyme activity site also possesses nucleic acid cleavage activity, the term ribozyme is used herein to include DNA, as long as it possesses sequence-specific nucleic acid cleavage activity. The most widely applicable ribozyme is self-splicing RNA found in infectious RNA of viroids, virusoids, etc.; such ribozymes include the hammerhead type and the hairpin type. The hammerhead type exhibits enzyme activity with about 40 bases, and is capable of specifically cleaving the target mRNA alone by rendering several bases at each end (about 10 bases in total) adjacent to the hammerhead structure complementary to the desired cleavage site of the mRNA. This type of ribozymes is also advantageous in that they do not attack genomic DNA because their substrate is RNA alone. When the GPRC5D mRNA by itself takes a double-stranded structure, it is possible to render the target sequence single-stranded by using a hybrid ribozyme resulting from the linking of an RNA motif from viral nucleic acid that is capable of specifically binding to RNA helicase [*Proc*. *Natl. Acad. Sci. USA, 98*(10) : 5572-5577 (2001)]. Furthermore, when using the ribozyme in the form of an expression vector containing DNA that encodes the ribozyme, the ribozyme may be a hybrid ribozyme resulting from the further linking of a sequence of modified tRNA to promote the transfer to cytoplasm [*Nucleic* Acids Res., 29(13): 2780-2788 (2001)].

Another embodiment of a substance that suppresses the expression of GPRC5D is a double-stranded oligo RNA that is complementary to a partial sequence in the coding region of the GPRC5D mRNA or its initial transcription product (including the intron portion in the case of the initial transcription product). What is called RNA interference (RNAi), a phenomenon wherein upon intracellular introduction of a short double-stranded RNA, an mRNA complementary to that RNA is decomposed, has long been known to occur in nematodes, insects, plants, and other organisms. Since this phenomena has recently been found to occur in animal cells as well [*Nature*, *411*(6836): 494-498 (2001)], RNAi is drawing attention for its potential as an alternative to ribozyme.

The antisense oligonucleotide and ribozyme of the present invention can be prepared by determining the target sequence for the mRNA or its initial transcription product on the basis of the GPRC5D cDNA sequence or genomic DNA sequence, and synthesizing a complementary sequence using a commercially available DNA/RNA autosynthesizer (Applied Biosystems, Beckman, etc.). A double-stranded oligo RNA possessing RNAi activity can be prepared by synthesizing a sense strand and an antisense strand respectively using a DNA/RNA autosynthesizer, denaturing each strand in the appropriate annealing buffer at about 90°C to about 95°C for about 1 minute, and subsequently annealing them at about 30°C to about 70°C for about 1 to about 8 hours. Additionally, a longer double-stranded polynucleotide can be prepared by synthesizing complementary oligonucleotide strands in alternative overlaps, annealing them, and subsequently subjecting them to ligation with ligase.

A preferred embodiment of a substance that suppresses the functional expression of GPRC5D at the post-translational level is an antibody against GPRC5D or a fragment thereof. This antibody may be a polyclonal antibody or monoclonal antibody, and can be prepared by a well-known immunological technique. Any fragment of the anti-GPRC5D antibody serves for the purpose, as long as it has an antigen-binding site (CDR) for GPRC5D, and is exemplified by Fab, F(ab')₂, ScFv, minibody, etc.

For example, a polyclonal antibody can be obtained by giving GPRC5D protein or a fragment thereof [may be prepared as a complex cross-linked with a carrier protein such as bovine serum albumin or KLH (Keyhole Limpet Hemocyanin), if necessary] as the antigen, along with a commercially available adjuvant (e.g., complete or incomplete Freund's adjuvant), to an animal by subcutaneous or intraperitoneal administration about 2 to 4 times at intervals of 2 to 3 weeks (the antibody titer of serum separated from drawn blood determined by a commonly known antigen-antibody reaction, and its elevation confirmed in advance), collecting whole blood about 3 to about 10 days after final immunization, and purifying the antiserum. Animals to be administered with the antigen include mammals such as rats, mice, rabbits, goat, guinea pigs and hamsters.

A monoclonal antibody can also be prepared by a cell fusion method (e.g., Takeshi Watanabe, *saibouyugouhou no genri to monokuronaru kotai no sakusei*, Akira Taniuchi and Toshitada Takahashi, eds., "*monokuronaru kotai to gan - kiso to rinsho-",* pp. 2-14, Science Forum Publishing, 1985). For example, a mouse is given this factor, along with a commercially available adjuvant, 2 to 4 times by subcutaneous or intraperitoneal administration, its spleen or lymph node is collected about 3 days after final administration, and leukocytes are separated. These leukocytes are fused with myeloma cells (e.g., NS-1, P3X63Ag8, etc.) to yield a hybridoma that produces a monoclonal antibody against this factor. The cell fusion may be achieved by the PEG method [*J*. *Immunol*. *Methods*, *81*(2): 223-228 (1985)] or the voltage pulsation method [*Hybridoma, 7*(6): 627-633 (1988)]. A hybridoma that produces the desired monoclonal antibody can be selected by detecting in the culture supernatant an antibody that specifically binds to an antigen using well-known EIA, RIA, or the like. Cultivation of a hybridoma that produces a monoclonal antibody can be conducted in vitro, or in vivo in mice or rats, preferably in ascites fluid of mouse, and the resulting antibody can be obtained from a hybridoma culture supernatant or animal ascites fluid, respectively.

However, in view of therapeutic effect and safety in humans, the anti-GPRC5D antibody of the present invention is preferably a chimeric antibody between a human and another animal (e.g., mice etc.), more preferably a humanized antibody. The term "chimeric antibody", as used herein, refers to an antibody having a variable region (V region) from an immunized animal and a constant region (C region) from a human; "humanized antibody" refers to an antibody wherein all regions except CDR have been replaced with a human antibody. A chimeric antibody or a humanized antibody can, for example, be obtained by cutting out a sequence that encodes a V region or CDR from the gene for a mouse monoclonal antibody prepared in the same manner as above, cloning a chimeric gene resulting from fusion with DNA that encodes a C region of an antibody from human myeloma into an appropriate expression vector, and introducing the vector to an appropriate host cell to express the chimeric gene.

Another preferred embodiment of a substance that suppresses the functional expression of GPRC5D at the post-translational level is an oligonucleotide that specifically binds to GPRC5D and inhibits its functional expression, i.e., aptamer. An aptamer for GPRC5D can, for example, be obtained by the procedure shown below. First, oligonucleotides (e.g., about 60 bases) are randomly synthesized using a DNA/RNA autosynthesizer to obtain a pool of oligonucleotides. Next, an oligonucleotide that binds to the protein of interest, i.e., GPRC5D is separated using an affinity column. The separated oligonucleotide is amplified by PCR and again screened through the aforementioned selection process. By repeating this process in about five cycles or more, an aptamer showing high affinity for GPRC5D can be selected.

A therapeutic agent for cibophobia comprising as an active ingredient a substance that suppresses the expression of GPRC5D is not capable of exhibiting its therapeutic activity unless it is incorporated in cells of the target tissue (i.e., hypothalamus); its active ingredient, nucleic acid or protein molecule, is not easily absorbable in cells and in addition is likely to undergo rapid decomposition in the body. Additionally, because the uptake of these molecules is usually carried out by endocytosis, they are likely to undergo decomposition by lysosome enzyme. Accordingly, it is important to design a drug delivery system (DDS) wherein a substance that suppresses the expression of GPRC5D is delivered to hypothalamic cells in a stable state so as to increase cell membrane permeability and to promote drug release from lysosome/endosome. For example, in the case of an oligo nucleic acid molecule such as an antisense oligonucleotide, it is possible to improve the stability to nuclease, intracellular transfer, and release from lysosome/endosome by chemical modifications such as PNA resulting from the replacement of the phosphate and sugar portions with peptide bonds, and oligonucleotides having a morphine backbone in place of a phosphate backbone, as well as nucleic acids with their phosphate linkage or sugar portion (2' position, 3' position, etc.) modified as described above; these modified oligo nucleic acids can easily be prepared using a DNA/RNA autosynthesizer.

On the other hand, cell membrane permeability can also be increased by coupling an accessory group such as poly-L-lysine, avidin, cholesterol or phospholipid to an oligonucleotide or antibody molecule.

Furthermore, it is also possible to prepare an oligonucleotide or antibody molecule as incorporated in a cationic liposome. By incorporating in a liposome, the active ingredient is protected against decomposition by nuclease and protease, and is incorporated in cells by endocytosis with the cationic surface of the liposome membrane binding to negatively charged molecules on the cell surface. Cationic liposome can, for example, be prepared by mixing a cationic lipid, such as DOTMA, DDAB or DMRIE, and DOPE, a neutral lipid capable of membrane fusion. Because nucleic acid and proteins are polyanionic, they easily form complexes when mixed with cationic liposomes. Additionally, it is possible to achieve cell-specific targeting by inserting in the liposome membrane an antibody or ligand for a cell surface molecule that is expressed specifically in hypothalamus cells. For example, the anti-GPRC5D antibody itself may be inserted in the liposome membrane.

Additionally, to protect the liposome incorporated by endocytosis against decomposition by lysosome enzyme, it is also preferable to use a pH-sensitive liposome (at acidic pH levels, the membrane becomes unstable and its contents are released from endosomic vesicles to cytoplasm before fusion with lysosome) or a liposome fused with Sendai virus wherein the viral RNA has been completely fragmented by ultraviolet irradiation etc. (the endocytosis pathway avoided by means of the membrane fusion capability of Sendai virus).

The therapeutic agent for cibophobia of the present invention designed in a dosage form as described above can be administered orally or parenterally by dissolving or suspending in an appropriate sterile vehicle. Examples of parenteral administration route include, for example, but are not limited to, systemic administrations such as intravenous, intra-arterial, intramuscular, intraperitoneal and intratracheal administrations, and local administration in the vicinity of the hypothalamus. Preferably, there may be mentioned local administration to the lateral ventricle.

The dosage of the therapeutic agent for cibophobia of the present invention varies depending on the kind of active ingredient, molecule size, administration route, severity of disease, animal species of administration subject, drug acceptability of administration subject, body weight, age, etc., and normally ranges from about 0. 0008 to about 2.5 mg/kg, preferably about 0.008 to about 0.025 mg/kg, based on the amount of active ingredient per day for each adult; such a dose may be administered at a time or in divided portions.

When the substance that suppresses the expression of GPRC5D is a nucleic acid molecule like an antisense nucleic acid, ribozyme or aptamer (hereinafter also referred to as effective nucleic acid molecule), the therapeutic agent for cibophobia of the present invention may comprise as an active ingredient an expression vector that encodes the effective nucleic acid molecule. With regard to the expression vector, oligonucleotide or polynucleotide that encodes the aforementioned effective nucleic acid molecule must be functionally linked to a promoter capable of exhibiting promoter activity in hypothalamus cells of the recipient mammal, or arranged at a position such that the oligonucleotide or polynucleotide is capable of turning to a form functionally linked to the promoter under particular conditions in hypothalamus cells of the administration animmal. Any promoter can be used, as long as it is capable of working in hypothalamus cells of the recipient mammal; such promoters include, for example, viral promoters such as the SV40-derived early promoter, cytomegalovirus LTR, Rous sarcoma virus LTR, MoMuLV-derived LTR and adenovirus-derived early promoter, and mammal constitutive protein gene promoters such as the β-actin gene promoter, PGK gene promoter and transferrin gene promoter. The wording "arranged at a position such that the oligonucleotide or polynucleotide is capable of turning to a form functionally linked to a promoter under particular conditions" means that, for example, the promoter and the oligo (poly) nucleotide that encodes an effective nucleic acid molecule are split by two recombinase recognition sequences arranged in the same direction, which are separated by a spacer sequence long enough to prevent expression of the effective nucleic acid molecule from the promoter, such that the spacer sequence is cleaved out in the presence of a recombinase that specifically recognizes the recognition sequence, thereby the polynucleotide that encodes the effective nucleic acid molecule is functionally linked to the promoter, as described in more detail below.

The expression vector of the present invention contains a transcription termination signal, i.e., a terminator region, preferably at downstream of an oligo(poly)nucleotide that encodes the effective nucleic acid molecule. Furthermore, the expression vector of the present invention may further contain selection marker genes for transformant selection (genes that confer resistance to such drugs as tetracycline, ampicillin, kanamycin, hygromycin and phosphinoslysine, genes that complement auxotrophic mutation, etc.). When the expression vector has a spacer sequence between recombinase recognition sequences as described above, the selection marker gene may also be arranged in the spacer sequence.

The vector used for the expression vector of the present invention is not particularly limitated; examples of vectors that are suitable for administration to mammals such as humans include viral vectors such as retrovirus, adenovirus, adeno-associated virus, herpes virus, vaccinia virus, pox virus, polio virus, Sindbis virus and Sendai virus. Adenovirus is advantageous in a number of features, including extremely high gene introduction efficiency and the capability of being introduced into non-dividing cells. It should be noted, however, that because the incorporation of the introduced gene into the host chromosome is extremely rare, this gene expression is transient and usually only lasts for about 4 weeks. In view of the persistence of the therapeutic effect, it is also preferable to use an adeno-associated virus, which is of relatively high gene introduction efficiency, which can be introduced to non-dividing cells as well, and which can be incorporated into chromosomes via an inverted terminal repeat sequence (ITR).

Effective nucleic acid molecules such as of antisense nucleic acids and ribozyme are in essence foreign substances; their constitutive and excess expression is highly toxic to the host animal introduced with gene and may cause adverse reactions. Accordingly, in a preferred embodiment of the present invention, the expression vector is capable of allowing an effective nucleic acid molecule to express time-specifically and/or hypothalamus cell-specifically to avoid the adverse effects of the excess expression of the effective nucleic acid molecule at an unwanted time and/or unwanted site. As a first example of such a vector, there may be mentioned a vector containing an oligo (poly) nucleotide that encodes an effective nucleic acid molecule linked functionally to a promoter derived from a gene specifically expressed in hypothalamus cells of the administration animal. There may be mentioned, for example, the native promoter of the GPRC5D gene.

As a second example of the time-specific and hypothalamus-specific expression vector of the present invention, there may be mentioned a vector containing an oligo (poly) nucleotide that encodes an effective nucleic acid molecule functionally linked to an inducible promoter which regulate an expressionby an exogenous substance in trans. For example, when using the metallothionein-1 gene promoter as the inducible promoter, the expression of the effective nucleic acid molecule can be induced hypothalamus-specifically at any time by locally administering an inducer such as a heavy metal, e.g., gold, zinc and cadmium, a steroid, e.g., dexamethasone, an alkylating agent, a chelating agent or a cytokine to the hypothalamus at the desired time.

Another preferred example of the time-specific and hypothalamus-specific expression vector of the present invention is a vector having the promoter and the oligo (poly) nucleotide that encodes an effective nucleic acid molecule which are split by two recombinase recognition sequences arranged in the same direction, wherein the recombinase recognition sequences are separated by a spacer sequence sufficiently long to prevent the expression of the effective nucleic acid molecule from the promoter. Solely introducing the vector in hypothalamus cells does not ensure that the promoter directs the transcription of the effective nucleic acid molecule. However, provided that a recombinase that specifically recognizes the recognition sequence is locally administered to the hypothalamus at the desired time, or an expression vector containing a polynucleotide that encodes recombinase is locally administered to express the recombinase in hypothalamus cells, homologous recombination via the recombinase occurs between the recognition sequences; as a result, the spacer sequence is cleaved out and the oligo(poly)nucleotide that encodes the effective nucleic acid molecule is functionally linked to the promoter, resulting in the hypothalamus-specific expression of the effective nucleic acid molecule at the desired time.

It is desirable that the recombinase recognition sequence used in the aforementioned vector should be a heterologous recombinase recognition sequence that is not recognized by endogenous recombinase so as to prevent the recombination by the recombinase present in the recipient. It is desirable, therefore, that the recombinase that act on the vector in trans should also be a heterologous recombinase. Preferred examples of such combinations of heterologous recombinase and the recombinase recognition sequence include, but are not limited to, a combination of Escherichia coli bacteriophage P1-derived Cre recombinase and the lox P sequence, and a combination of yeast-derived Flp recombinase and the frt sequence.

Discovered in a bacteriophage, Cre recombinase is known to work in the specific DNA recombination reaction, not only in prokaryotic cells but also in animal cells which are eukaryotic cells and animal viruses. When two lox P sequences are present on the same DNA molecule in the same direction, Cre recombinase cleaves out the DNA sequence between the sequences to allow them to form a cyclic molecule (cleavage reaction). On the other hand, in cases where two lox P sequences are present on different DNA molecules one of which is cyclic DNA, the cyclic DNA is inserted to the other DNA molecule via the lox P sequence (insertion reaction) [*J*. *Mol*. *Biol*., *150*: 467-486 (1981) ; *J. Biol. Chem*., *259*: 1509-1514 (1984); *Proc. Natl. Acad. Sci. USA, 81*: 1026-1029 (1984)]. Example cleavage reactions are reported in cultured animal cells [*Nucleic Acids Res.*, *17*: 147-161 (1989); *Gene*, *181*: 207-212 (1996)], animal viruses [*Proc*. *Natl. Acad. Sci. USA, 85*: 5166-5170 (1988); *J. Virol*., *69*: 4600-4606 (1995); *Nucleic Acids Res*., *23*: 3816-3821 (1995)], transgenic mice [*Proc. Natl. Acad. Sci. USA, 89*: 6232-6236 (1992); *Proc*. *Natl*. *Acad. Sci. USA, 89*: 6861-6865 (1992); *Cell, 73*: 1155-1164 (1993); *Science, 265*:103-106 (1994)], etc.

As the promoter for the time-specific and hypothalamus-specific expression vector of the present invention, which is based on the interaction of a recombinase/recombinase recognition sequence, there may preferably be used a virus-derived promoter or a mammal constitutive protein gene promoter to ensure the expression at the desired time and site.

Administration of the therapeutic agent for cibophobia of the present invention comprising an expression vector that encodes an effective nucleic acid molecule as an active ingredient is carried out by either the *ex vivo* method, in which nerve cells of the animal to be treated are taken out from the body, cultured, then returned to the body by introduction, and the *in vivo* method, in which the vector is introduced by directly administering it to the recipient's body. In case of the *ex vivo* method, introduction of the vector into the target cell can be carried out by the microinjection method, calcium phosphate co-precipitation method, PEG method, electroporation method, etc. In case of the *in vivo* method, the viral vector is administered in the form of an injection or the like intravenously, intra-arterially, subcutaneously, intracutaneously, intramuscularly, intraperitoneally or the like. Alternatively, administering a vector by intravenous injection etc. may pose a problem with the production of a neutralizing antibody against the viral vector; however, it is possible to mitigate the adverse effects of the presence of the antibody by locally injecting the vector in the vicinity of the hypothalamus, where the target cell is present, e.g., in the lateral ventricle (*in situ* method).

Additionally, when using a non-viral vector as the expression vector that encodes an effective nucleic acid molecule, introduction of the expression vector can be carried out by use of a high molecular carrier such as a poly-L-lysine-nucleic acid complex or liposome encapsulation as described above with respect to dosage forms of therapeutic drugs comprising the effective nucleic acid molecule itself as an active ingredient. Alternatively, it is also possible to introduce the vector directly to the target cell using the particle gun method.

When recombinase itself is locally administered as the trans-acting substance in the use of a vector based on recombinase/recombinase recognition sequence interaction, recombinase, for example, may be injected to the hypothalamus on dissolving or suspending in an appropriate sterile vehicle (e.g., artificial cerebrospinal fluid etc.). On the other hand, when a recombinase expression vector is locally administered to the hypothalamus as the trans-acting substance, the recombinase expression vector may be any vector, as long as it possesses an expression cassette having the recombinase-encoding polynucleotide functionally linked to a promoter capable of exhibiting promoter activity in hypothalamus cells of the administration subject. When the promoter used is a constitutive promoter, it is desirable that the vector administered to the hypothalamus for preventing the expression of recombinase at unwanted times should be a vector that rarely undergoes incorporation in the host cell chromosome, e.g., adenovirus. However, when using an adenovirus vector, the transient expression of recombinase persists for about 4 weeks at most; if the treatment is prolonged, a second or third administration will be necessary. As another approach to expressing a recombinase at the desired time, there may be mentioned the use of an inducible promoter like the metallothionein gene promoter. In this case, viral vectors of high integration efficiency such as retrovirus can be used.

When the substance that suppresses the expression of GPRC5D is a nucleic acid molecule like an antisense nucleic acid, ribozyme or aptamer, the therapeutic agent for cibophobia of the present invention may contain as an active ingredient a host cell containing an expression vector that encodes an effective nucleic acid molecule as described above. As examples of useful host cells, there may be mentioned autologous cells taken out as target cells from the recipient in the aforementioned *ex vivo* introduction method for an expression vector, nerve cells taken out from allogenic (e.g., stillborn fetuses, brain death patients, etc., in case of humans) or heterologous (non-human mammals such as swine and simian, in case of humans) individuals, or nerve cells obtained by culturing and differentiating such nerve stem cells or ES cells. Because the central nervous system is the organ/tissue where rejection is most unlikely, even heterologous cells can be allowed to take using a small amount of immunosuppressant in combination.

In another embodiment, it is possible to transform a resident bacterium in the nasal cavity, throat, oral cavity, intestine, or the like of the recipient animal as the host cell, with an expression vector that encodes an effective nucleic acid molecule by a conventional method, and to deliver the thus-obtained transformant to a site of the recipient where the host cell normally occurs. In recent years, a route other than the blood-brain barrier route has been investigated via which a drug is transferred from the nose directly to the cerebrospinal fluid for delivery to the brain; the use of a nasal cavity resident bacterium suffices that objective.

The dosage of the therapeutic agent for cibophobia of the present invention comprising, as an active ingredient, an expression vector that encodes an effective nucleic acid molecule or a host cell harboring the expression vector varies depending on the kind of active ingredient, molecule size, promoter activity, administration route, severity of disease, animal species of administration subject, drug acceptability of administration subject, body weight, age, etc., and is preferably at a level that causes expression of an effective nucleic acid molecule in an amount equivalent to an appropriate dosage of a therapeutic drug comprising the effective nucleic acid molecule itself as an active ingredient in the body of an animal that has received a vector or host cell, and is exemplified by about 2 to about 20 µg/kg, preferably about 5 to about 10 µg/kg based on the amount of vector per day for each adult.

Because GPRC5D is a membrane receptor protein that mediates signal transduction for negatively regulating food consumption, food intake behavior can be suppressed by enhancing the expression of this receptor. Accordingly, the present invention also provides a therapeutic agent for a lifestyle-related disease comprising a substance that enhances the expression of GPRC5D as an active ingredient. In general, the "lifestyle-related disease" is defined as a group of diseases whrein lifestyles such as dietary habits, exercise habits, resting, smoking and drinking are responsible for the onset and progress thereof. The same term, as used herein, specifically refers to "a group of diseases wherein a therapeutic effect can be achieved by adjusting food intake to reduce it," typically exemplified by diabetes, obesity, hyperlipidemia, hyperuricemia, etc. Patients often have two or more of these diseases at a time.

Examples of substances that enhance the expression of GPRC5D include trans-acting factors capable of promoting RNA transcription from the GPRC5D gene, factors capable of promoting splicing or mRNA transfer to cytoplasm, factors that suppress mRNA decomposition, factors capable of promoting binding of ribosome to mRNA, factors that suppress the decomposition of the GPRC5D protein, and factors that promote the transport of the GPRC5D protein to the membrane; as preferred examples of more directly acting specific substances, there may be mentioned the GPRC5D protein or an equivalent thereof, an expression vector containing a nucleic acid that encodes the GPRC5D, or a host cell harboring the expression vector.

The "GPRC5D protein" as used herein is a protein consisting of the amino acid sequence shown by SEQ ID NO:2 or SEQ ID NO:4; "an equivalent of GPRC5D protein" refers to a polypeptide consisting of an amino acid sequence resulting from the substitution, deletion, insertion, addition or modification of 1 or more (preferably 1 to 50, more preferably 1 to 30, still more preferably 1 to 10, and most preferably 1 to 5) amino acids in the amino acid sequence shown by SEQ ID NO:2 or SEQ ID NO:4, that exhibits a ligand-receptor interaction equivalent to that of a protein consisting of the amino acid sequence shown by SEQ ID NO: 2 or SEQ ID NO:4, and that couples with Gα to promote the GDP-GTP exchange reaction of the subunit or, being outside the range of this definition, a protein derived from a different mammal, i.e. orthlog, which is encoded by a gene having the same molecular evolution origin as human or mouse GPRC5D gene. Accordingly, the therapeutic agent for a lifestyle-related disease of the present invention is intended for use to treat diabetes, obesity, hyperlipidemia, hyperuricemia, etc., not only in humans or mice but also in other mammals. Because the number of animals suffering from diseases like lifestyle-related diseases, such as obesity due to excess feeding and a lack of exercise, has been increasing with the recent pet animal boom, the remedy of the present invention is very useful in the field of veterinary medicine as well.

The GPRC5D protein or an equivalent thereof can be isolated from a membrane-containing fraction derived from the hypothalamus tissue of humans or mice, or other mammals such as bovine, swine, simian or rat by affinity chromatography with the anti-GPRC5D antibody. Alternatively, a DNA clone isolated from a cDNA library or genomic library derived from the tissue with the GPRC5D cDNA clone as a probe can be cloned into an appropriate expression vector, introduced to the host cell, expressed, and purified from the membrane-containing fraction of the cell culture by affinity chromatography with the anti-GPRC5D antibody or His-tag, GST-tag or the like. The equivalent may be partially introduced a mutation by an artificial treatment such as site-directed mutagenesis based on the GPRC5D cDNA sequence (the base sequence shown by base numbers 1-1035 in the base sequence shown by SEQ ID NO: 1 or base numbers 148-1047 in the base sequence shown by SEQ ID NO:3). Conservative amino acid substitution is well known; those skilled in the art can introduce a mutation as appropriate in the GPRC5D protein, as long as the receptor characteristics of GPRC5D remain unchanged. However, because the ligand binding domain and preferably the extracellular loop to which an inverse agonist is capable of binding, and the N-terminal strand must be conserved to high extents, it is desirable that a mutation should not be introduced in such regions.

A therapeutic agent for a lifestyle-related disease comprising the GPRC5D protein or an equivalent thereof as an active ingredient can be modified to increase its cell membrane permeability by coupling an accessory group such as poly-L-lysine, avidin, cholesterol or phospholipid component as described above with respect to a therapeutic agent for cibophobia comprising the anti-GPRC5D antibody as an active ingredient. Alternatively, this therapeutic agent can also be prepared by encapsulating the GPRC5D protein or an equivalent thereof into a cationic liposome. Because proteins are poly-anionic, the protein easily forms a complex when mixed with a cationic liposome. Additionally, it is also possible to achieve cell-specific targeting by incorporating into the liposome membrane an antibody or ligand for a cell surface molecule specifically expressed in hypothalamus cells. For example, it is also possible to incorporate the anti-GPRC5D antibody (preferably an antibody not having antagonist activity or inverse agonist activity) to the liposome membrane.

A therapeutic agent for a lifestyle-related disease comprisng the GPRC5D protein or an equivalent thereof as an active ingredient can be administered orally or parenterally on dissolving or suspending in an appropriate sterile vehicle. Examples of parenteral administration route include, for example, but are not limited to, systemic administrations such as intravenous, intra-arterial, intramuscular, intraperitoneal and intratracheal administrations, and local administration in the vicinity of the hypothalamus. Preferably, there may be mentioned local administration to the lateral ventricle.

The dosage of the present therapeutic agent for a lifestyle-related disease varies depending on the administration route, severity of disease, animal species of administration subject, drug acceptability of administration subject, body weight, age, etc., and normally ranges from about 0.0008 to about 2.5 mg/kg, preferably about 0.008 to about 0.025 mg/kg based on the amount of active ingredient per day for each adult; such a dose may be administered at a time or in divided portions.

When the substance that enhances the expression of GPRC5D is the GPRC5D protein or an equivalent thereof, the therapeutic agent for a lifestyle-related disease of the present invention may be an expression vector containing a nucleic acid that encodes such a polypeptide, or may be a host cell harboring the expression vector. The expression vector and host cell used here may be identical to those used for the aforementioned cibophobia remedy. Furthermore, regarding the administration route and dosage for these lifestyle-related disease remedies, those exemplified above with respect to cibophobia remedies can be used preferably.

The present invention also provides a therapeutic agent for cibophobia comprising as an active ingredient a substance that suppresses the function of GPRC5D expressed on the cell membrane of hypothalamus cells, or a therapeutic agent for a lifestyle-related disease comprising as an active ingredient a substance that promotes such function. These therapeutic agents can be obtained by screening for substances that exhibit agonist activity, antagonist activity or inverse agonist activity to GPRC5D. Accordingly, the present invention also provides at a time a screening method for a substance that suppresses or promotes the function of GPRC5D, and a screening system for the same method.

The term "agonist activity", as used herein, refers to a property by which the substance in question specifically binds to the GPRC5D receptor and causes a shift of the equilibration between the active and inactive forms of GPRC5D toward the active side. Accordingly, substances having agonist activity include physiological ligands for GPRC5D, as well as what is called full agonists and partial agonists. The term "antagonist activity" refers to a property by which the substance in question competitively binds to the ligand-binding site of GPRC5D but has no or almost no effect on the equilibration between the active and inactive forms. Accordingly, substances having antagonist activity are understood to be what is called neutral antagonists, and not to include inverse agonists. The term "inverse agonist activity" refers to a property by which the substance in question binds to any site of GPRC5D and causes a shift of the equilibration between the active and inactive forms of GPRC5D toward the inactive side. The simple term "ligand" as used herein, is understood to include all physiological ligands, agonists, antagonists and inverse agonists.

The screening system of the present invention is a series of receptor-G protein co-expression systems obtained by constructing a constituent unit for the receptor-binding region of each Gα family (i.e., Gαₛ, Gαᵢ, Gα_{q}), which constituent unit consists of a lipid bilayer membrane containing the GPRC5D protein or an equivalent thereof, and a polypeptide comprising at least the receptor-binding region of a Gα belonging to a family and the guanine nucleotide-binding region of any Gα, as essential member constituents. The GPRC5D protein or an equivalent thereof is identical to that mentioned above as an active ingredient of the aforementioned therapeutic agent for a lifestyle-related disease. Although the lipid bilayer membrane retaining the GPRC5D protein or an equivalent thereof may be of any origin, as long as the receptor protein is allowed to take the essential steric structure on the membrane, it is preferably exemplified by fractions containing the cell membrane of eukaryotic cells such as human, bovine, swine, simian, mouse, rat or other mammal cells, and insect cells, e.g., intact cells, cell homogenates, or cell membrane fractions fractionated from these homogenates by centrifugation etc.. However, an artificial lipid bilayer membrane prepared by a conventional method from a solution of various lipids, e.g., phosphatidylcholine, phosphatidylserine, and cholesterol, mixed at an appropriate ratio, preferably a ratio close to abundance ratios in the cell membranes of eukaryotic cells such as mammal cells and insect cells, can also be used preferably in an embodiment of the present invention.

Gα (Gαₛ), belonging to the Gₛ family, promotes the activity of adenylate cyclase as the effector, and is exemplified by Gαₛ₋₁-Gαₛ₋₄ and G_{olf}. Gα (Gαᵢ), belonging to the Gᵢ family, suppresses the activity of adenylate cyclase as the effector, and is exemplified by Gαᵢ₋₁-Gαᵢ₋₃ and G_{z}. Gα (Gα_{q}), belonging to the G_{q} family, promotes the activity of phospholipase C as the effector, and is exemplified by Gα_{q} and G₁₆. The Gαₛ polypeptide, Gαᵢ polypeptide and Gα_{q} polypeptide of the present screening system need to have a region involved in the binding to its own GPCR (RB region) and a region involved in the binding to any guanine nucleotide of Gα (GB region). Results of X-ray crystallographic analysis of Gα have shown that GPCR-binding region is located in the vicinity of the C-terminus whereas the GB region is a region homologous to the nucleotide-binding site of the ras protein (from the N-terminus side: amino acid motives called the P box, G' box, G box, and G" box, the leader of the αE helix in a highly helical domain, αF helix, etc.). When a physiological ligand or agonist to GPRC5D binds to the receptor, the Gα activation domain of the receptor and the Gα RB region that couples with the receptor interact with each other to produce a conformational change in Gα, resulting in the dissociation of GDP from the GB region and quick binding of GTP. Gα-GTP acts on the effector to promote or suppress its activity. On the other hand, binding an inverse agonist inactivates the Gα active domain due to a conformational change in the receptor, resulting in a decreased active Gα-GTP level and inhibition of its action on the effector. Here, provided that a GTP analogue that does not undergo hydrolysis by the GTPase activity of Gα, such as ³⁵S-labeled GTPγS, has been added to the system in place of GTP, it is possible to evaluate the effects of the test substance on the GDP-GTP exchange reaction in Gα by determining and comparing the radioactivity bound to the membrane in the presence and absence of the test substance, and to screen for substances that possess ligand activity for GPRC5D. Hence, provided that the radioactivity has increased in the presence of a test substance, the test substance can be judged to possess agonist activity to GPRC5D and hence therapeutic activity for lifestyle-related disease. Conversely, provided that the radioactivity has decreased, the test substance can be judged to possess inverse agonist activity to GPRC5D and hence therapeutic activity for cibophobia.

Once ligands for GPRC5D are screened for, a family that couples with the receptor is elucidated; subsequent screening can be conducted using only a system containing a Gα polypeptide belonging to the family as a member constituent. The results described below of constitutive activation of GPRC5D using a receptor-Gα fusion protein expression system strongly suggest that the G protein α subunit capable of coupling with GPRC5D may be Gαₛ. Accordingly, the present invention also provides a screening method for ligands for the receptor characterized in that the GDP-GTP exchange reaction of the Gα or the activity of the effector that interacts with the Gα is compared in the presence and absence of the test substance in a co-expression system of a G protein α subunit (preferably Gαₛ) capable of coupling with GPRC5D and the receptor.

The activity of a ligand for GPRC5D can also be determined with the action of a Gα polypeptide on the effector as an index. In this case, the screening system of the present invention must further comprise as another constituent a lipid bilayer membrane containing the effector, in addition to the GPRC5D protein or an equivalent thereof. Additionally, the Gα polypeptide must further comprise a region for interaction with the effector. Because the Gα members belonging to the individual families differ with respect to the kind of effector or the direction of action, it is preferable that all Gα polypeptides share the effector interaction region, rather than each has its own effector interaction region. Accordingly, in the present screening system, at least two kinds of Gα polypeptides are chimeric polypeptides containing the effector interaction region of a Gα belonging to another family. For example, when using phospholipase C as the effector, the Gα_{q} polypeptide may be a native one; however, the Gαₛ polypeptide and Gαᵢ polypeptide must be chimeric polypeptides wherein the effector interaction region has been replaced with that of Gα_{q}. As the simplest example of a chimeric polypeptide containing the effector interaction region of a Gα belonging to another family, there may be mentioned a chimeric polypeptide wherein about 5 amino acids at the C-terminus of a Gα belonging to another family (i.e., RB region) have been replaced with its own C-terminal sequence.

In the present screening system, when three kinds of Gα polypeptides contain an effector interaction region of Gαₛ or Gαᵢ, a lipid bilayer membrane containing adenylate cyclase is used as the effector. On the other hand, when the Gα polypeptide contains the effector interaction region of Gα_{q}, a lipid bilayer membrane containing phospholipase C must be used as the effector.

In a screening system comprising adenylate cyclase (hereinafter also referred to as AC) as the effector, the action of a Gα polypeptide on the effector can be evaluated by directly determining the AC activity. The AC activity can be determined using any commonly known technique; examples of useful methods include, but are not limited to, a method comprising adding ATP to an AC-containing membrane fraction, and determining the resulting cAMP content by competitive immunoassay using an anti-cAMP antibody in the presence of cAMP labeled with a RI (e.g., ¹²⁵I), an enzyme (e.g., alkaline phosphatase, peroxidase, etc.), a fluorescent substance (e.g., FITC, rhodamine, etc.), or the like, and a method comprising adding [α-³²P] ATP to an AC-containing membrane, separating the resulting [³²P] cAMP using an alumina column etc., and subsequently determining the radioactivity thereof. When the Gα polypeptide contains the effector interaction region of Gαₛ, and determining and comparing the AC activity in the presence and absence of a test substance, provided that the AC activity has increased in the presence of the test substance, the test substance can be judged to possess agonist activity to GPRC5D and hence therapeutic activity for lifestyle-related disease. Conversely, provided that the AC activity has decreased, the test substance can be judged to possess inverse agonist activity to GPRC5D and hence therapeutic activity for cibophobia. On the other hand, when the Gα polypeptide contains the effector interaction region of Gα, provided that the AC activity has increased in the presence of a test substance, the test substance can be judged to possess inverse agonist activity to GPRC5D and hence therapeutic activity for cibophobia. Conversely, provided that the AC activity has decreased, the test substance can be judged to possess agonist activity to GPRC5D and hence therapeutic activity for lifestyle-related disease.

When using an intact eukaryotic cell as the screening system, the action of a Gα polypeptide on AC can also be evaluated by determining the intracellular cAMP content, or labeling the cell with [³H] adenine, and determining the radioactivity of resulting [³H] cAMP. Although the intracellular cAMP content can be determined by incubating cells in the presence and absence of the test substance for an appropriate time, subsequently disrupting the cells, and subjecting the thus-obtained extract by the aforementioned competitive immunoassay, any other commonly known method can be used.

In another embodiment, the cAMP content may be evaluated by determining the amount of expression of reporter gene under the control of the cAMP-response element (CRE). The expression vector used here is described in detail below, and is outlined below. The intracellular cAMP content is determined by culturing a eukaryotic cell incorporating a vector containing an expression cassette with a DNA that encodes the reporter protein linked downstream of a CRE-containing promoter, in the presence and absence of the test substance for an appropriate time, disrupting the cells, and measuring and comparing the expression of the reporter gene in the thus-obtained extract using a commonly known technique.

Accordingly, when the Gα polypeptide contains the effector interaction region of Gas, provided that the intracellular cAMP content (or the amount of expression of reporter gene under the control of CRE) has increased in the presence of a test substance, the test substance can be judged to possess agonist activity to GPRC5D and hence therapeutic activity for lifestyle-related disease. Conversely, provided that the cAMP content (or the amount of expression of reporter gene) has decreased, the test substance can be judged to possess inverse agonist activity to GPRC5D and hence therapeutic activity for cibophobia. On the other hand, when the Gα polypeptide contains the effector interaction region of Gαᵢ, provided that the intracellular cAMP content (or the amount of expression of reporter gene under the control of CRE) has increased in the presence of a test substance, the test substance can be judged to possess inverse agonist activity to GPRC5D and hence therapeutic activity for cibophobia. Conversely, provided that the cAMP content (or the amount of expression of reporter gene) has decreased, the test substance can be judged to possess agonist activity to GPC5D and hence therapeutic activity for lifestyle-related disease.

On the other hand, in a screening system containing phospholipase C (hereinafter also referred to as PLC) as the effector (i.e., a case wherein the Gα polypeptide contains the effector interaction region of Gα_{q}), the action of the Gα polypeptide on the effector can be evaluated by directly determining the PLC activity. The PLC activity can, for example, be evaluated by adding ³H-labeled phosphatidylinositol 4,5-diphosphate to a PLC-containing membrane fraction, and determining the amount of inositol phosphate produced using a commonly known technique. The PLC activity is determined and compared in the presence and absence of the test substance; provided that the PLC activity has increased in the presence of the test substance, the test substance can be judged to possess agonist activity to GPRC5D and hence therapeutic activity for lifestyle-related disease. Conversely, provided that the PLC activity has decreased, the test substance can be judged to possess inverse agonist activity to GPRC5D and hence therapeutic activity for cibophobia.

When using an intact eukaryotic cell as the screening system, the action of Gα polypeptide on PLC can also be evaluated by adding [³H] inositol to the cell and determining the radioactivity of the resulting [³H] inositol phosphate, or determining the intracellular Ca²⁺ content. Although the intracellular Ca²⁺ content can be determined by spectroscopy using a fluorescent probe (fura-2, indo-1, fluo-3, Calcium-Green I, etc.) or by using a calcium-sensitive luminescent protein such as aequorin after the cells are incubated for a given time in the presence and absence of the test substance, any other commonly known method can be used. As an apparatus suitable for spectroscopy using a fluorescent probe, there may be mentioned the FLIPR (Molecular Devices Company) system.

In another embodiment, the Ca²⁺ content may be evaluated by determining the amount of expression of reporter gene under the control of Ca²⁺-upregulated TPA (12-O-tetradecanoylphorbol-13-acetate)-respondse element (TRE). The expression vector used in that method is described in detail below, and is outlined below. The intracellular Ca²⁺ content is determined by culturing a eukaryotic cell incorporating a vector containing an expression cassette with a DNA that encodes the reporter protein linked downstream of a TRE-containing promoter, in the presence and absence of the test substance for an appropriate time, disrupting the cells, and measuring and comparing the expression of the reporter gene in the thus-obtained extract using a commonly known technique.

Accordingly, provided that the intracellular Ca²⁺ content (or the amount of expression of reporter gene under the control of TRE) has increased in the presence of a test substance, the test substance can be judged to possess agonist activity to GPRC5D and hence therapeutic activity for lifestyle-related disease. Conversely, provided that the intracellular Ca²⁺ content (or the amount of expression of reporter gene) has decreased, the test substance can be judged to possess inverse agonist activity to GPRC5D and hence therapeutic activity for cibophobia.

The substance subjected to the screening method of the present invention may be any commonly known compound or a new compound, and is exemplified by compound libraries prepared using combinatorial chemistry techniques, random peptide libraries prepared by solid phase synthesis or the phage display method, and naturally occurring components such as those derived from microorganisms, animals, plants, and marine organisms.

A preferred embodiment of a system containing as essential constituents a lipid bilayer membrane containing the GPRC5D protein or an equivalent thereof, and Gα polypeptide, which system is a constitution unit of the screening system of the present invention, is a host eukaryotic cell transfected with both an expression vector containing a DNA that encodes the GPRC5D protein or an equivalent thereof and an expression vector containing a DNA that encodes a polypeptide at least comprising the RB region of a Gα belonging to a family and the GB region of any Gα, a homogenate of the cell, or a membrane fraction from the cell.

The "DNA that encodes the GPRC5D protein or an equivalent thereof" is not particularly limited, as long as it is a DNA that encodes a polypeptide consisting of the amino acid sequence shown by SEQ ID NO:2 or SEQ ID NO:4, or a polypeptide that consists of an amino acid sequence resulting from the substitution, deletion, insertion, addition or modification of 1 or more (preferably 1 to 50, more preferably 1 to 30, still more preferably 1 to 10, and most preferably 1 to 5) amino acids in the amino acid sequence shown by SEQ ID NO:2 or SEQ ID NO:4, that exhibits a ligand-receptor interaction equivalent to that of GPRC5D, and that couples with Gα to promote the GDP-GTP exchange reaction of the subunit or a DNA that encodes an ortholog of a polypeptide consisting of the amino acid sequence shown by SEQ ID NO:2 or SEQ ID NO:4. As such DNAs, there may be mentioned, for example the GPRC5D cDNA coding region (the base sequence shown by base numbers 1-1035 in the base sequence shown by SEQ ID NO:1 or the base sequence shown by base numbers 148-1047 in the base sequence shown by SEQ ID NO:3), as well as DNAs that encodes a GPCR corresponding to GPRC5D of non-human or mouse mammal origin such as of bovine, swine, simian, or rat; these can be isolated from cDNA libraries or genomic libraries derived from cerebral nerve tissue, including the mammal hypothalamus using the GPRC5D cDNA as a probe. The equivalent may partially incorporate a mutation introduced by an artificial treatment such as site-directed mutagenesis based on the GPRC5D cDNA.

The DNAs that encode the three kinds of Gα polypeptides needs to have at least a sequence that encodes the RB region of the Gα in each family, and a sequence that encodes the GB region of any Gα. The sequences of the various Gα genes are commonly known and the RB region and GB region are well known from the results of X-ray crystallographic analysis of Gα as described above. Accordingly, those skilled in the art can easily construct a fragment lacking a portion of the coding sequence of Gα as desired.

In a screening system based on the action of Gα polypeptide on the effector as an index, the DNA that encodes the Gα polypeptide must further contain a nucleic acid sequence that encodes the effector interaction region. Because the three kinds of Gα polypeptides share the effector interaction region as described above, at least two kinds of Gα polypeptides are chimeras having the effector interaction region of different families. As the simplest example of a DNA that encodes the chimeric polypeptide, there may be mentioned a chimeric polypeptide wherein cDNA sequence encoding about 5 amino acids at the C-terminus of a Gα containing the desired effector interaction region have been replaced with a DNA sequence that encodes the C-terminal sequence of a Gα belonging to another family.

The DNA that encodes the GPRC5D protein or an equivalent thereof and the DNA that encodes the Gα polypeptide must be functionally linked to a promoter capable of exhibiting promoter activity in the host eukaryotic cell. Any promoter can be used, as long as it is capable of working in eukaryotic cell; such promoters include, for example, viral promoters such as the SV40-derived early promoter, cytomegalovirus LTR, Rous sarcoma virus LTR, MoMuLV-derived LTR and adenovirus-derived early promoter, and eukaryote-derived constitutive protein gene promoters such as the β-actin gene promoter, PGK gene promoter and transferrin gene promoter. It is preferable that the expression vector used contain in addition to the aforementioned promoter a transcription termination signal, i.e., a terminator region, downstream thereof, and it is desirable that the expression vector has an appropriate restriction endonuclease recognition site, preferably a unique restriction endonuclease recognition site that cleaves the vector only at one position, so that a coding DNA can be inserted between the promoter region and the terminator region. Furthermore, the expression vector may further contain a selection marker gene (drug resistance genes such as for tetracycline, ampicillin, kanamycin, hygromycin and phosphinothricin, auxotrophic mutation complementary genes, etc.).

As examples of vectors useful in the screening system of the present invention, there may be mentioned plasmid vectors, viral vectors that are suitable for use in mammals such as humans, including retrovirus, adenovirus, adeno-associated virus, herpes virus, vaccinia virus, pox virus, polio virus, Sindbis virus and Sendai virus, and vaculovirus vectors that are suitable for use in insect cells.

The DNA that encodes the GPRC5D protein or an equivalent thereof and the DNA that encodes the Gα polypeptide may be co-transfected to the host cell as carried on two separate expression vectors, or introduced to the host cell as inserted in a single vector dicistronically or monocistronically.

The host cell may be any one, as long as it is a eukaryotic cell such as a mammal cell such as a human, simian, mouse, rat or hamster cell, or an insect cell. Specifically, such host cells include mouse-derived cells such as COP, L, C127, Sp2/0, NS-1, NIH3T3 and ST2, rat-derived cells, hamster-derived cells such as BHK and CHO, simian-derived cells such as COS1, COS3, COS7, CV1 and Vero, and human-derived cells such as HeLa and 293, as well as insect-derived cells such as Sf9, Sf21 and High Five.

Gene introduction to the host cell can be achieved using any commonly known method applicable to gene introduction to eukaryotic cells; examples of such methods include the calcium phosphate co-precipitation method, the electroporation method, the liposome method, and the microinjection method.

The gene-incorporating host cell can, for example, be cultured using a minimum essential medium (MEM) containing about 5% to about 20% bovine fetal serum, Dulbecco's modified Eagle medium (DMEM), RPMI1640 medium, 199 medium, etc. Medium pH is preferably about 6 to about 8; culturing temperature is normally about 27°C to about 40°C.

The thus-obtained eukaryotic cell incorporating a DNA that encodes the GPRC5D protein or an equivalent thereof and a DNA that encodes a Gα polypeptide may be used as an intact cell as is, depending on the screening method used, or may be used in the form of a cell homogenate obtained by disrupting the cell in an appropriate buffer solution, or a membrane fraction isolated by centrifuging the homogenate under appropriate conditions (e.g., supernatant recovered via centrifugation at about 1,000×g, followed by centrifugation at about 100,000×g and recovery of the precipitation).

For example, when the ligand characteristics of the test substance are evaluated by GTPγS-binding assay or direct determination of the effector activity, the screening system used is preferably a membrane fraction prepared from cells as described above. On the other hand, when the ligand characteristics of the test substance are evaluated by determining the intracellular cAMP content (or the amount of expression of cAMP-response reporter) or intracellular Ca²⁺ content (or the amount of expression of Ca²⁺-response reporter), the screening system used is an intact eukaryotic cell.

When evaluating ligand activity with the amount of expression of a cAMP-responding reporter (in cases where the effector is adenylate cyclase) or Ca²⁺-responding reporter (in cases where the effector is phospholipase C) as an index, the host eukaryotic cell must incorporate a vector containing an expression cassette wherein a DNA that encodes the reporter protein is functionally linked downstream of a promoter region containing a cAMP-responding element (CRE) or TPA-responding element (TRE). CRE is a cis-element that activates gene transcription in the presence of cAMP, exemplified by a sequence containing TGACGTCA as a consensus sequence, and may be a sequence containing a deletion, substitution, insertion or addition, as long as cAMP responsiveness is retained. On the other hand, TRE is a cis-element that activates gene transcription in the presence of Ca²⁺, exemplified by a sequence containing TGACTCA as a consensus sequence, and may be a sequence containing a deletion, substitution, insertion or addition, as long as Ca²⁺ responsiveness is retained. As a CRE- or TRE-containing promoter sequence, there may be used in the same manner virus promoters and eukaryotic cell constitutive protein gene promoters as described above; using a restriction enzyme and DNA ligase, or by means of PCR etc., the CRE or TRE sequence can be inserted downstream of the promoter sequence. As the reporter gene under the control of CRE or TRE, there may be used any commonly known gene that permits quick and simple detection and quantitation of the expression thereof; such genes include, for example, but are not limited to, DNAs that encode such reporter proteins as luciferase, β-galactosidase, β-glucuronidase, alkaline phosphatase and peroxidase. More preferably, a terminator sequence is arranged downstream of the reporter gene. As such a vector carrying a CRE (or TRE)-reporter expression cassette, there may be used a commonly known plasmid vector or viral vector.

Another preferred example of a system containing as essential constituents a lipid bilayer membrane containing the GPRC5D protein or an equivalent thereof, and a Gα polypeptide, which system is a constitution unit of the screening system of the present invention, is a host eukaryotic cell transfected with an expression vector containing a DNA that encodes a fused protein wherein a polypeptide at least comprising the RB region of a Gα belonging to a family and the GB region of any Gα is linked to the C-terminus side of the GPRC5D protein or an equivalent thereof, a homogenate of the cell, or a membrane fraction from the cell.

A DNA encoding GPRC5D protein or an equivalent thereof, and a DNA encoding a polypeptide containing an RB-binding region of Gα of each family and a GB region of any Gα can be obtained as mentioned above. Those of ordinary skilled in the art can easily construct a DNA encoding a fused protein of GPRC5D and Gα polypeptide by appropriately combining known genetic engineering methods based on these DNA sequences. For example, a method comprising ligating a DNA encoding GPRC5D, whose termination codon has been deleted, with a DNA encoding GPRC5D to match reading frame using PCR and the like can be mentioned. In this case, deleting a part of C-terminal of GPRC5D and inserting a linker sequence such as His-tag between GPRC5D and Gα are also possible.

A DNA encoding the obtained fused protein is inserted into an expression vector as mentioned above, and introduced into an eucaryotic host cell by the above-mentioned gene introduction technique. When the fused protein is expressed on the obtained eucaryotic cell membrane, and when GPRC5D and Gα can interact, Gα active domain on intracellular loop 3 of the receptor and RB region of Gα interact in the absence of a physiological ligand for the receptor, and can promote the GDP/GTP exchange reaction in Gα. In other words, Gα stays constitutively being activated. In contrast, a fused protein with Gα that does not interact with GPRC5D does not activate GPRC5D, and does not increase Gα-GTP level. Here, when a GTP analog free of hydrolysis by GTPase activity of Gα, such as ³⁵S-labeled GTPγS, is added to the system instead of GTP, activation of GPRC5D can be evaluated by measuring the radioactivity bound with the membrane and comparing with each other in the membrane systems respectively containing three kinds of fused proteins, thereby identifying the Gα capable of interacting with the receptor.

Once a Gα capable of interactng with GPRC5D is identified, the subsequent screening can be conducted using only a membrane system containing GPRC5D and the Gα, preferably only a membrane system containing a fused protein of GPRC5D and Gα. In other words, in the same manner as in the identification of the above-mentioned coupled Gα, the effect of a test substance on the GDP/GTP exchange reaction in Gα can be evaluated by adding, to the system, a GTP analog free of hydrolysis due to GTPase activity of Gα, and measuring and comparing the radioactivities bound with the membrane in the presence and absence of the test substance, and a substance having a GPCR ligand activity can be screened for. When the radioactivity increases in the presence of a test substance, the test substance has an agonist activity to GPRC5D, and when the radioactivity decreases, the test substance has an inverse agonist activity to GPRC5D. Since a receptor is activated only partially by a fused protein, when a physiological ligand or an agonist to GPRC5D is bound, the activity-non-activity balance of the receptor shifts toward the active side, and the Gα-GTP level increases further. Thus, this screening system can screen for agonists as well.

As shown in the Examples below, because GPRC5D is constitutively activated only when expressed as a fused protein with Gas, Gα coupled with the receptor is strongly suggested to be Gαₛ. Therefore, the present invention also provides a screening method for a ligand for the receptor, which comprises comparing, in a fused protein expression system of Gαₛ and the receptor, a GDP/GTP exchange reaction of Gαₛ in the presence and absence of the test substance.

Activation of GPRC5D in a fused protein can be also evaluated using, as an index, an action of Gα on an effector. In this case, the screening system of the present invention needs to be a membrane system encompassing, in addition to each fused protein, a lipid bilayer further containing an effector each Gα interacts with. That is, a membrane system containing a fused protein with Gα_{q} further contains phospholipase C (PLC), a membrane system containing a fused protein with Gαᵢ and Gαₛ further contains adenylate cyclase (AC). In this case, the presence or absence of activation of GPRC5D can be also evaluated by preparing, for each Gα, a membrane system containing GPRC5D and Gα separately (that is, not as a fused protein), and measuring and comparing the activity of effector (that is, in a membrane system containing a fused protein of GPRC5D and Gα capable of interaction, the activity of effector is significantly high (low in the case of Gαᵢ) as compared to a membrane system containing the both as non-fused proteins, and for those that do not interact, there is no significant difference in the activity of effector between the both systems).

Once a Gα capable of interacting with GPRC5D is identified, the subsequent screening can be conducted using only a membrane system containing a fused protein with said Gα by directly or indirectly measuring and comparing the activity of an effector the Gα can interact with, in the presence and absence of the test substance. Therefore, the present invention also provides a screening method for a ligand for GPRC5D, which comprises measuring and comparing, in a membrane system containing a fused protein of Gα capable of being coupled with a receptor identified by the above-mentioned identification method of Gα coupled with GPRC5D and the receptor and an effector with which said Gα is capable of interaction, the activity of an effector, in the presence and absence of the test substance.

As mentioned above, because GPRC5D is constitutively activated only when expressed as a fused protein with Gαₛ, Gα coupled with the receptor is strongly suggested to be Gαₛ. Therefore, the AC activity in a membrane system containing a fused protein of GPRC5D and Gαₛ is measured and compared in the presence and absence of the test substance. The AC activity can be measured in the same manner as in the above.

It is also known that Gα can be constitutively activated by introducing a mutation by a known method into a specific part of a DNA that encodes Gα and modifying the amino acid sequence thereof. Accordingly, this system can be used similarly for screening for a ligand. Such technique can be performed according to the method described in, for example, *Mol. Pharmacol*., *57*, 890-898 (2000) and *Biochemistry, 37,* 8253-8261 (1998).

For such fused protein (or mutant Gα) expression cell, too, any form of intact cell, cell homogenate and membrane fraction can be appropriately selected and used according to the screening method to be employed.

In another embodiment of the present invention, as a screening system containing, as constituent elements, a lipid bilayer membrane containing GPRC5D protein or an equivalent thereof, and Gα polypeptide, one obtained by re-constituting purified GPRC5D protein or an equivalent thereof with Gα polypeptide, or a purified fused protein of the receptor with Gα, in an artificial lipid bilayer membrane can be used. The GPRC5D protein or an equivalent thereof can be purified by affinity chromatography with the use of anti-GPRC5D antibody and the like from membrane fraction obtained from cerebral nerve tissue and the like including hypothalamus of human or mouse, or other mammals. Alternatively, the receptor can be purified by affinity chromatography using anti-GPRC5D antibody, His-tag, GST-tag and the like, from a recombinat cell into which an expression vector containing a DNA encoding GPRC5D protein or an equivalent thereof has been introduced. Similarly, a fused protein of the receptor and Gα can be also purified by affinity chromatography using anti-GPRC5D antibody, His-tag, GST-tag and the like, from a recombinat cell into which an expression vector containing a DNA encoding the fused protein has been introduced.

As a lipid composing an artificial lipid bilayer membrane, phosphatidyl choline (PC), phosphatidyl serine (PS), cholesterol (Ch), phosphatidyl inositol (PI), phosphatidyl ethanolamine (PE) and the like can be mentioned. A mixture of one or more kinds thereof mixed at a suitable ratio is preferably used.

For example, an artificial lipid bilayer membrane (proteoliposome) incorporating a receptor and Gα or a receptor-Gα fused protein can be prepared by the following methods. First, a suitable amount of a mixed lipid chloroform solution of PC:PI:Ch=12:12:1 is separated in a glass tube, chloroform is evaporated in a nitrogen gas vapor to dry the lipid in the form of a film, a suitable buffer is added to suspend the lipid, which is uniformly dispersed by ultrasonication, a buffer containing a surfactant such as sodium cholate and the like is further added to completely suspend the lipid. Thereto is added a suitable amount of purified receptor and Gα, or a receptor-Gα fused protein, and after incubation for about 20-30 min while sometimes stirring in ice water, dialyzed against a suitable buffer, centrifuged at about 100,000xg for 30-60 min and the precipitation is recovered to give a desired proteoliposome.

A substance having a therapeutic activity against cibophobia or a lifestyle-related disease, which is selected by a screening system or a screening method as mentioned above can be prepared into a therapeutic agent for cibophobia or a lifestyle-related disease by combining any pharmaceutically acceptable carrier.

Accordingly, the present invention provides a therapeutic agent for cibophobia, which comprises as an active ingredient an antagonist or an inverse agonist to GPRC5D, which is selected by the screening method of the present invention. The present invention also provides a therapeutic agent for a lifestyle-related disease, which comprises as an active ingredient a physiological ligand or agonist to GPRC5D, which is selected by the screening method of the present invention.

The pharmaceutically acceptable carrier is exemplified by, but not limited to, excipients such as sucrose, starch, mannit, sorbit, lactose, glucose, cellulose, talc, calcium phosphate, calcium carbonate and the like, binders such as cellulose, methylcellulose, hydroxypropylcellulose, polypropylpyrrolidone, gelatine, gum arabic, polyethylene glycol, sucrose, starch and the like, disintegrating agents such as starch, carboxymethyl cellulose, hydroxypropyl starch, sodium-glycol-starch, sodium hydrogen carbonate, calcium phosphate, calcium citrate and the like, lubricants such as magnesium stearate, aerosil, talc, sodium lauryl sulfate and the like, aromatics such as citric acid, menthol, glycyl lysine ammonium salt, glycine, orange powder and the like, preservatives such as sodium benzoate, sodium bisulfite, methylparaben, propylparaben and the like, stabilizers such as citric acid, sodium citrate, acetic acid and the like, suspending agents such as methylcellulose, polyvinylpyrrolidone, aluminum stearate and the like, dispersing agents such as surfactant and the like, diluents such as water, physiological saline, orange juice and the like, base wax such as cacao butter, polyethylene glycol, refined kerosene and the like, and the like.

A preparation which is suitable for oral administration is, for example, a liquid comprising an effective amount of a ligand dissolved in a diluent such as water, physiological saline and orange juice, a capsule, sachet or tablet comprising an effective amount of a ligand as a solid or granules, a suspension comprising an effective amount of a ligand in a suitable dispersion medium, an emulsion comprising a solution of an effective amount of a ligand dispersed and emulsified in a suitable dispersion medium and the like.

A preparation preferable for parenteral administration (e.g., subcutaneous injection, intramuscular injection, topical injection, intraperitoneal administration and the like) includes, for example, an aqueous or non-aqueous isotonic sterile injection which may contain antioxidant, buffer, bacteriostatic agent, isotonicity agent and the like. It may be an aqueous or non-aqueous sterile suspension which may contain suspension, solubilizer, thickener, stabilizer, preservative and the like. When the administration method is topical injection near the hypothalamus, an injection containing ligand as an active ingredient dissolved or suspended in an artificial cerebrospinal fluid is preferable. Alternatively, it can be formulated into a sustained release preparation using a biocompatible material such as collagen and the like. Since pluronic gel gelates at body temperature and becomes a liquid at a lower temperature, long duration can be afforded by topically injecting the ligand along with pluronic gel to allow for gelation around the target tissue. The ligand preparation can be sealed in a container in a unit dose or plural doses like an ampoule or vial. It is also possible to lyophilize a ligand and a pharmaceutically acceptable carrier and preserve them in a state that only requires dissolving or suspending in a suitable sterile vehicle immediately before use.

While the dose of the ligand preparation of the present invention varies depending on ligand activity (full agonist or partial agonist, or an antagonist or inverse agonist), degree of seriousness of the disease, the animal species to be the administration subject, drug acceptability, body weight and age of the administration subject, and the like, it is generally about 0.0008 - about 2.5 mg/kg, preferably about 0.008 - about 0.025 mg/kg, a day for an adult in the amount of the ligand.

The present invention is explained in more detail by referring to Examples, which are mere exemplification and not to be construed as limitative. Unless particularly specified, the following examples were performed according to the methods described in Sambrook, J. et al., Molecular Cloning: A Laboratory Manual, 2nd ed., Cold Spring Harbor Laboratory Press, New York (1989), Current Protocols in Protein Science (ed. Coligan, J. E. et al.), John Wiley and Sons, Inc. and the like.

### Reference example 1: Analysis of expression distribution of GPRCSD gene by RT-PCR

The hypothalamus from db/db mouse (male, SPF grade, 7-week-old at the time of purchase, 10-week-old at the time of organ sampling, CLEA JAPAN, INC.) was homogenized in Trizol (Gibco), and total RNA was obtained. cDNA was synthesized using RNA PCR Kit (AMV) Ver2.1. (Takara) and the total RNA (1 µg) as a template. PCR was performed using the obtained cDNA from hypothalamus as a template and the following mouse GPRC5D (mGprc5d) specific primer. As a Taq polymerase, one from Perkin Elmer, Inc. was used.

5'-ACC TCT TCT GTG ACA ACG AG-3' (SEQ ID NO: 5)

5'-GGA AGA GGA CAT AGA TCA GG-3' (SEQ ID NO: 6)

### As a result, expression of GPRC5D gene in the hypothalamus of db/db mouse was clearly shown.

### Reference example 2: Analysis of expression distribution of GPRC5D gene by in situ hybridization

After perfusion fixation with 10% neutral buffered formalin, the brain was taken from mouse (CD-1 (ICR)) and after fixation with 10% neutral buffered formalin, the block was prepared by paraffin embedding. The paraffin block was sliced in a 7µm-thick coronal section (in the vicinity of Interaural 2.34 mm, Bregma-1.46 mm), and used as a section for ISH staining.

In addition, digoxigenin-labeled RNA probe was prepared by an In vitro transcription method with T3 and T7RNA polymerase. Specifically, antisense RNA probe corresponding to the region of 234^{th} - 624^{th} of SEQ ID NO: 3 was prepared using DIG RNA Labeling Mix (Roche).

Next, using mouse brain paraffin section and probe prepared above, ISH staining was performed by a conventional method. Using anti-digoxigenin antibody labeled with alkaline phosphatase as antibody and NBT/BCIP as coloring substrate, nuclear staining with Kernechtrot was performed after staining.

As a result of staining using a GPRC5D antisense probe, expression in nucleus paraventricularis and hypothalamus ventromedial nucleus was found. The nucleus paraventricularis and hypothalamus ventromedial nucleus are the regions where the feeding center exists. Thus, GPRC5D was suggested to be a factor involved in the feeding and energy metabolism in the feeding center.

### Example 1: Action of GPRC5D clone antisense DNA on obese model mouse

### (1) Experimental materials

Reagent: As a GPRC5D clone antisense, a 25 mer thiolized antisense DNA was used, which corresponded to the vicinity of initiation codon of the gene. The base sequences of the antisense DNA are shown in the following.

5′-TCA TAC ATG GTG ACT TAT AGG TAG A-3′ (SEQ ID NO: 7)

As a control DNA, thiolized oligo DNA having the following sequence was used.

5′-CCT CTT ACC TCA GTT ACA ATT TAT A-3′ (SEQ ID NO: 8)

This sequence is complimentary to the sequence produced by the mutation which causes a splicing abnormality at position 705 in pre-mRNA of erythrocyte β-globin in hemoglobinopathy thalassemia. It is assumed that the oligosaccharide has no specific target region or biological activities in normal cells and corrects the splicing abnormality only when acted on erythrocyte of thalassemia, thereby producing normal β-globin coding mRNA. The synthesis, thiolation and HPLC purification of these oligo DNAs were committed to Nihon Bio Service Co., Ltd.

For other reagents, commercially available special grade reagents were used.
Experimental animal: C57BL db/db (hereinafter, referred as obese mouse) (SPF grade) were purchased from CLEA JAPAN, INC., and after preliminary breeding, the obese mice were used for the test at the age of 10 weeks.
Breeding environment: The mice were bred in a room controlled to a temperature of not lower than 20°C and not higher than 26°C, relative humidity of not less than 30% and not more than 70%, lighting cycle of 8:00-20:00 lighting and 20:00-8:00 lights-out. During breeding, the mice were allowed a free access to a solid feed (CE-2, CLEA JAPAN) and sterile distilled water.

### (2) Preparation of administration liquid

As an antisense DNA administration liquid and a control DNA administration liquid, an artificial cerebrospinal fluid (0.166 g/L CaCl₂, 7.014 g/L NaCl, 0.298 g/L KCl, 0.203 g/L MgCl₂·6H₂O, 2.10 g/L NaHCO₃) containing 7.5 µg/µl of antisense DNA was prepared.

### (3) Administration of antisense DNA in cerebral ventricle

The obese mice were divided into two groups, and after fasting overnight, an antisense DNA administration liquid was given at 4 µl/mouse (30 µg/mouse for antisense DNA) to one group and a control DNA administration liquid (4 µl/mouse) was administered into the lateral ventricle of the other group, simultaneously with the lighting at 8:00 a.m..

### (4) Effect of antisense DNA administration on food consumption and blood glucose level of mice

Feeding of mice was resumed immediately after administration, and food consumption was calculated every 12 hours up to 48 hours after administration. The effect of the antisense DNA on food consumption of obese mice is shown in Fig. 1. In Fig.1, black columns show average food consumption at every 12 hours after administration to control DNA administration group and white columns show that of antisense DNA administration group (error bar is standard deviation, control DNA administration groups n=4, antisense DNA administration groups n=4). There was no big change in the amount of food consumption up to 12 hours after administration. As the time advances, however, administration of the antisense DNA of GPRC5D clone showed an effect of increased food consumption in obese mice.

Additionally, blood glucose level was measured before administration and every 24 hours up to 48 hours after administration. The effect of antisense DNA on blood glucose level of obese mice is shown in Fig. 2. In Fig.2, black columns show average blood glucose level at every 24 hours after administration of control DNA administration groups and white columns show that of antisense DNA administration groups (error bar is standard deviation, control DNA administration groups n=4, antisense DNA administration groups n=4). There was no big change in the blood glucose level up to 24 hours after administration. At 48 hours after administration, however, administration of the antisense DNA of GPRC5D clone showed an effect of increased blood glucose level in obese mice.

From the above-mentioned results, the possibility was suggested that GPRC5D clone is a factor involved in feeding behavior and glucose metabolism.

### Example 2: Analysis of variation in hypothalamus gene expression in satiation and fasting states

Hypothalamus was taken from db/db mouse (male, SPF grade, 7-week-old at the time of purchase, 11-week-old at the time of organ sampling, CLEA JAPAN, INC.; hereinafter to be referred to as obese mouse) and C57BL/6N mouse (male, SPF grade, 6-week-old at the time of purchase, 11-week-old at the time of organ sampling, Charles River, Japan, Inc.; hereinafter to be reffered to as nomal mouse) in satiation state or 24 hour fasting state, homogenated in Trizol (Gibco) and total RNA was obtained. Reverse transcription reaction was performed using 1 µg of total RNA and SYBR Green RT-PCR Reagent (Roche). Subsequently, each sample after reverse transcription reaction and GPRC5D clone specific primer (see below) were mixed, PCR reaction was performed using ABI7700 sequence detector (PE biosystems) and variation in the expression of gene of GPRC5D clone was analyzed. In addition, GPRC5D clone expression was calibrated with GAPDH (glyceraldehyde-3-phosphate dehydrogenase) and analyzed as relative values.

### ·GPRC5D

forward primer:
5′-GGA GTA TCT CAT CCC ATC AGC TAC A-3′ (SEQ ID NO: 9)

reverse primer:
5'-CAC TCT TCC AGG TTG TCT CTA TGC-3' (SEQ ID NO: 10)

### ·GAPDH

forward primer:
5′-CAA GAG AGG CCC TAT CCC AAC T-3′ (SEQ ID NO: 11)

reverse primer:
5′-CTA GGC CCC TCC TGT TAT TAT GG-3′ (SEQ ID NO: 12)

Variation in expression of GPRC5D gene in the satiation/fasting state is shown in Fig. 3. In Fig. 3, black columns show average variation in the expression of GPRC5D gene in satiation state, white columns show that in the expression of GPRC5D gene in the fasting state (error bar shows standard deviation, n=3 in each groups). There was found no big variation in normal mouse. On the contrary, promoted expression of GPRC5D gene was found in obese mouse in the fasting state. The promoted expression of GPRC5D clone in obese mouse in the fasting state and promoted food consumption and increased blood glucose level due to the administration of antisense suggested the possibility of GPRC5D clone having a food consumption suppressive action. From the above findings, it has been clarified that GPRC5D clone is involved in the regulation of food consumption and glucose metabolism, and that promotion of the action of GPRC5D clone provides a more effective therapeutic effect on lifestyle-related diseases.

### Example 3: Establishment of GPRC5D stable expression cell line

### (1) Construction of transgene vector

The coding region of GPRC5D clone is amplified by PCR method using KOD-Plus (TOYOBO). The amplified gene fragment is inserted into pcDNA3.1 (Invitrogen) and a GPRC5D expression vector is constructed.

### (2) Establishment of cell line

CHO cell lines expressing three types of chimeric G proteins (Gq, Gqi5, Gqs5) are seeded in 10 cm² culture dishes and cultured in a D-MEM medium (Gibco) containing 10% FBS (Gibco) until 60-70% confluent. Then, the cells are transferred to a serum-free medium, complexed with the GPRC5D expression vector constructed in the above (1) and Lipoofectamine-Plus (Gibco), and added to the medium. After incubation for 5 hours, the medium is changed to D-MEM medium containing 10% FBS and the cells are further cultured for 8 hours. Then cells are detached from the petri dish with trypsin-EDTA, suspended in D-MEM medium containing 500 µg/ml G418 and 10% FBS and seeded in 10 cm² petri dishes. Few days later, formed colonies are isolated and named as GPRC5D stable expression cell lines (¥q, ¥qi5, ¥qs5).

### Example 4: Screening for receptor agonist

### (1) Preparation of cells

GPRC5D stable expression cell lines (¥q, ¥qi5, ¥qs5) are seeded in 96-well culture dishes and cultured in a D-MEM medium (Gibco) containing 10% FBS (Gibco) until 60-70% confluent. They are used as expression cells.

### (2) Addition of reagents

The medium of expression cells is changed to serum free D-MEM medium one day before use. On assessment day, each compound (2.5 mM DMSO solution) is diluted to desired concentrations with D-MEM medium. Medium in the culture dishes is removed, and the diluted test compound, 4 µM Fluo3-AM (Teflab.) and 2.5 mM probenecid are added and it is cultured at 37°C for 60 min. A sample treated in the same way except that the test compound is not added is prepared for comparison.

### (3) Measurement of intracellular calcium concentrations by FLIPR method

The cells treated as mentioned above are washed with ice-cooled PBS, and suspended in Thyrode's medium (containing 2.5 mM probenecid and 1% gelatin). Absorbance of culture dish at 488 nM and 540 nM is quantified by FLIPR (Molecular Devices).

### Example 5: Construction of plasmid for expression of GPRC5D-Gα fused protein

As three kinds of representative α subunits considered to almost cover all GPCR signaling, Gα16 was selected from the Gq family, Gαi2 was selected from the Gi family and GαS2 was selected from the Gs family. All these coding regions were PCR cloned into expression vector pcDNA3.1(+). A restriction enzyme EcoRV cleavage site and a sequence containing 6×His tags were added just before each Gα coding region by PCR, whereby plasmids pcHISGα16, pcHISGαi2 and pcHISGαS2 capable of easy fusion with GPCR gene on the 5' side of each Gα protein were prepared.

That is, human spleen cDNA (Clontech) was diluted 20 times, 1 µl thereof was used as a template for amplification. As the enzyme, employed was KODplus (TOYOBO). However, the Mg²⁺ concentration was 1 mM. For amplification reaction, 20 µl of a reaction solution was used, which was a reaction buffer attached with KODplus. To amplify Gα16 cDNA, primer GNA15F1 (SEQ ID NO: 13) and GNA15R1 (SEQ ID NO: 14) were used; to amplify Gαi2 cDNA, primer GNAi2F1 (SEQ ID NO: 15) and GNAi2R1 (SEQ ID NO: 16) were used; and to amplify GaS2 cDNA, primer GS2F1 (SEQ ID NO: 17) and GS2R1 (SEQ ID NO: 18) were used. For amplification, GeneAmp PCR System 9600 (Applied Biosystems) was used and amplification was conducted under the conditions of 94°C, 2 min → (94°C, 15 sec → 68°C, 120 sec) × 40 cycles to give a PCR product having an object size. The terminal of each PCR product was phosphorylated, separated and purified by 0.8% agarose gel electrophoresis, cleaved with restriction enzyme EcoRV, ligated with plasmid pcDNA3.1(+)(Invitrogen) treated with CIAP and used to transform Escherichia coli DH5α. Plasmids were purified from the transformant line, and upon confirmation that the restriction enzyme digestion pattern and inserted base sequence were objective ones, the obtained G protein expression plasmids were named pcGα16, pcGαi2 and pcGaS2, respectively.

To fuse GPRC5D via HIS tag on N terminal of each G protein, HIS tag sequence was ligated immediately before initiation codon ATG of each G protein, and a restriction enzyme EcoRV recognition site was further added. That is, PCR was conducted using 10 ng each of pcGα16, pcGαi2 and pcGaS2 as a template, a primer (GNA15ATG; SEQ ID NO: 19, GNAi2ATG; SEQ ID NO: 20 or GS2ATG; SEQ ID NO: 21) and a primer (pcDNARV; SEQ ID NO: 22) of vector. Amplification was conducted under the conditions of 94°C, 2 min → (94°C, 15 sec → 58°C, 30 sec → 68°C, 60 sec) × 20 cycles to give a PCR product having the object size. Then HIS2 linker (SEQ ID NO: 23) and HIS2 linker (R) (SEQ ID NO: 24) were annealed, and after phosphorylation of the terminal, ligated with each PCR product. These were digested with restriction enzymes EcoRV and XhoI, and the object DNA fragments were separated and purified by 0.8% agarose gel electrophoresis. The recovered DNA fragments were ligated with expression plasmid pcDNA3.1 (+) digested with EcoRV and XhoI, and used to transform *Escherichia coli* DH5α. Plasmids were purified from transformant line, and upon confirmation that the restriction enzyme digestion pattern and inserted base sequence were object ones, the obtained plasmids were named pcHISGα16, pcHISGαi2 and pcHISGαS2, respectively.

### Example 6: Construction of GPRC5D-Gα fused protein expression plasmid

### (1) cloning of GPRC5D

PCR cloning of GPRC5D gene was performed with human testis derived cDNA library (clontech) and the following primers.

5'-GGAGAAGGGCATCAGAAAAC-3' (SEQ ID NO: 25)

5'-TTATACTCCTCCTGCATCTTGC-3' (SEQ ID NO: 26)

The obtained fragment was subjected to TA-cloning with pT7Blue vector (Novagen). The plasmid was prepared from the obtained clone, and analyzed for the base sequence. As a result, GPRC5D gene having a sequence identical to the sequences of published references was obtained. The obtained plasmid was named as pT7GPRC5D.

### (2) construction of GPRC5D expression vector

Construction of expression vector was carried out using GATEWAY system of Invitrogen Corp. and in accordance with the vendor's instructions. First, expression vectors were converted to destination vectors. pcHISGαS2, pcHISGαi2 and pcHISGα16 prepared in example 5 were each digested with EcoRV then GATEWAY frame B (Invitrogen) was inserted. DB3.1 compitent cells (Invitrogen) after transformation were selected with chloramphenicol, the obtained clones were further selected by digesting with restriction enzymes to give desired clones having GATEWAY frame B insert in the right direction. These were named as pcHisGαS2-DEST, pcHisGαi2-DEST and pcHisGα16-DEST, respectively. In addition, a destination vector having the same CMV promoter and bGH terminator was used as a non-fused gene expression vector for control. Specifically, pcDNA3.1mycHisA was digested with Hind III-Xba I and then blunted, and GATEWAY frame C.1 (Invitrogen) was inserted. Then DB3.1 compitent cells (Invitrogen) after transformation were selected with chloramphenicol, the obtained clones were further selected by digesting with restriction enzymes, and a clone having GATEWAY frame C.1 insert in the right direction was obtained, which was named as pcDNA3.1-DEST.

Next, using pT7GPRC5D as a template and the following primers, ORF (R-type) having up to intact stop codon, and ORF (F-type) with only intact stop codon deleted were amplified by PCR. After electrophoresis and purification by cutting out from the gel, these ORFs were subjected to BP clonase (Invitrogen) reaction to be newly carried on donor vector pDONR201.

atGPRC5DM (forward primer):
5'-GGGGACAAGTTTGTACAAAAAAGCAGGCTCCACCatgtacaaggactgcatcg
agtcc-3' (SEQ ID NO: 27)

atGPRC5DR (reverse primer for R-type):
5'-GGGGACCACTTTGTACAAGAAAGCTGGGTCAttatactcctcctgcatcttgc
tg-3' (SEQ ID NO: 28)

atGPRC5DF (reverse primer for F-type):
5'-GGGGACCACTTTGTACAAGAAAGCTGGGTCtactcctcctgcatcttgctg-3
' (SEQ ID NO: 29)

By analysis of the base sequence of the obtained clones, desired clones were each confirmed to have been obtained. The obtained clones were named as pENTR/GPRC5D/R (R-type entry clone) and pENTR/GPRC5D/F (F-type entry clone), respectively.

By LR clonase reaction of these, pENTR/GPRC5D/F was crossed over 3 kinds of Gα fused destination vectors (pcHisGαS2-DEST, pcHisGαi2-DEST and pcHisGα16-DEST) and pENTR/GPRC5D/R was crossed over pcDNA3.1-DEST. In these obtained clones, plasmids were prepared on a small scale and the desired clone was selected by restriction enzyme treatment. The obtained plasmids were named as pcC5D/His/GαS2, pcC5D/His/Gαi2, pcC5D/His/Gα16 and pcC5D, respectively. Further, using Qiagen Maxi Kit, mass preparation and purification of plasmid were done from 100 ml of culture fluid with the aim of introduction into cell lines.

### Example 7: Confirmation of constitutive activation by expression of GPRC5D-Gα fused protein

To examine whether constitutive activation occurs by cellular expression a protein with Gα protein fused at the c-terminal of GPRC5D, the following experiment was carried out.

1 µg of DNA for four kinds of plasmid vectors (pcC5D, pcC5D/His/GαS2, pcC5D/His/Gαi2 and pcC5D/His/Gα16) constructed in Example 6, and pcDNA3.1 (+) (Invitrogen: Cat. No. V790-20) were diluted with 125 µl of OPTI-MEM I medium (Invitrogen: Cat. No. 31985-062) (solution A). 2.5 µl of Lipofectamine 2000 Reagent (Invitrogen: Cat. No. 1168-027) which is a transfection reagent, were diluted with 125 µl of OPTI-MEM I medium and stood for 5 minutes (solution B). Solution A and solution B were mixed and incubated for 20 minutes, and then added at 50 µg/well (triplicate) to Chinese Hamster Ovary cells (CHO-K1 cells, ATCC No. CCL-61) plated 3 x 10⁴ cells/well the day before. After culture at 37°C for 4 hours under condition of 5% CO₂, the medium was changed to F12 medium (Invitrogen: Cat. No. 11765-054) containing 10% FCS at 100 µl/well and further cultured for about 12 hours. Intracellular cAMP levels of these cells were measured using HitHunter™ EFC Cyclic AMP Chemiluminescens Assay Kit (Applied Biosystem: Cat. No. DRX-0027), cAMP measurement kit, and in accordance with the attached protocol (Fig. 4). As a result, cAMP levels were confirmed to have been specifically increased by fusing GPRC5D with GαS2, suggesting that GPRC5D can be constitutively activated by fusing with GαS2. Therefore, the GPRC5D was strongly suggested to be a GPCR that is coupled with GαS.

### Sequence Listing Free Text

SEQ ID NO: 5: Oligonucleotide designed to function as a primer to amplify mRNA of GPRC5D.
SEQ ID NO: 6: Oligonucleotide designed to function as a primer to amplify mRNA of GPRC5D.
SEQ ID NO: 7: Oligonucleotide designed to function as an antisense DNA inhibiting expression of GPRC5D.
SEQ ID NO: 8: Oligonucleotide designed to function as an antisense DNA for sequence resulted from mutation causing abnormal splicing at position 705 of β-globin pre-mRNA in thalassemia.
SEQ ID NO: 9: Oligonucleotide designed to function as a primer to amplify mRNA of GPRC5D.
SEQ ID NO: 10: Oligonucleotide designed to function as a primer to amplify mRNA of GPRC5D.
SEQ ID NO: 11: Oligonucleotide designed to function as a primer to amplify mRNA of GAPDH.
SEQ ID NO: 12: Oligonucleotide designed to function as a primer to amplify mRNA of GAPDH.
SEQ ID NO: 13: Oligonucleotide designed to function as a sense primer to amplify human G protein Gα16 cDNA fragment containing full length ORF.
SEQ ID NO: 14: Oligonucleotide designed to function as an antisense primer to amplify human G protein Gα16 cDNA fragment containing full length ORF.
SEQ ID NO: 15: Oligonucleotide designed to function as a sense primer to amplify human G protein Gαi2 cDNA fragment containing full length ORF.
SEQ ID NO: 16: Oligonucleotide designed to function as an antisense primer to amplify human G protein Gαi2 cDNA fragment containing full length ORF.
SEQ ID NO: 17: Oligonucleotide designed to function as a sense primer to amplify human G protein GαS2 cDNA fragment containing full length ORF.
SEQ ID NO: 18: Oligonucleotide designed to function as an antisense primer to amplify human G protein GαS2 cDNA fragment containing full length ORF.
SEQ ID NO: 19: Oligonucleotide designed to function as a sense primer to amplify human G protein Gα16 cDNA fragment from the initiation codon.
SEQ ID NO: 20: Oligonucleotide designed to function as a sense primer to amplify human G protein Gαi2 cDNA fragment from the initiation codon.
SEQ ID NO: 21: Oligonucleotide designed to function as a sense primer to amplify human G protein GαS2 cDNA fragment from the initiation codon.
SEQ ID NO: 22: Oligonucleotide designed to function as an antisense primer to amplify multicloning sites of plasmid pcDNA3.1(+).
SEQ ID NO: 23: Sense chain oligonucleotide designed to construct a linker containing a nucleotide sequence encoding a 6xHis tag peptide sequence.
SEQ ID NO: 24: Antisense chain oligonucleotide designed to construct a linker containing a nucleotide sequence encoding 6xHis tag peptide sequence.
SEQ ID NO: 25: Oligonucleotide designed to function as a sense primer to amplify cDNA of GPRC5D.
SEQ ID NO: 26: Oligonucleotide designed to function as an antisense primer to amplify cDNA of GPRC5D.
SEQ ID NO: 27: Oligonucleotide designed to function as a sense primer to amplify ORF of cDNA of GPRC5D.
SEQ ID NO: 28: Oligonucleotide designed to function as an antisense primer to amplify ORF(R-type) of cDNA of GPRC5D.
SEQ ID NO: 29: Oligonucleotide designed to function as an antisense primer to amplify ORF(F-type) of cDNA of GPRC5D.

### Industrial Applicability

Since GPRC5D is a GPCR involved in feeding behavior, the pharmaceutical composition of the present invention containing, as an active ingredient, a substance that enhances or suppresses expression or function of GPRC5D can regulate food intake to a desired level and is expected to afford a therapeutic effect on lifestyle-related diseases caused by overeating, such as diabetes, obesity, hyperlipidemia and the like, or cibophobia. According to the screening system and screening method of the present invention, moreover, a ligand for GPRC5D can be easily and rapidly screened for and they are useful for the development of a new drug targeting GPRC5D, search for a disease marker and establishment of a diagnostic method using the disease marker.

While the present invention has been described with an emphasis on preferred embodiments, it will be obvious to those of ordinary skilled in the art that variations of the preferred embodiments may be used. It is intended that the invention may be practiced otherwise than as specifically described herein. Accordingly, this invention includes all modifications encompassed within the spirit and scope of the invention as defined by the following claims.

This application is based on a patent application No. 397523/2001 filed in Japan, the contents of which are hereby incorporated by reference. The references cited herein, including patents and patent applications, are hereby incorporated in their entireties by reference, to the extent that they have been disclosed herein.

## Claims

1. A therapeutic agent for cibophobia comprising, as an active ingredient, a substance that suppresses expression or function of a polypeptide consisting of an amino acid sequence shown in SEQ ID NO: 2 or SEQ ID NO:4.

2. A therapeutic agent for cibophobia comprising, as an active ingredient, a nucleic acid of the following (a) or (b):
(a) a nucleic acid consisting of a base sequence complementary to a base sequence shown in SEQ ID NO: 1 or SEQ ID NO: 3
(b) a nucleic acid consisting of a base sequence capable of hybridizing with a nucleic acid consisting of a base sequence shown in SEQ ID NO: 1 or SEQ ID NO: 3 or a primary transcript which generates said base sequence after post-transcriptional processing under physiological conditions of hypothalamus of a subj ect animal for treatment, and which is capable of inhibiting translation into a polypeptide encoded by the base sequence shown in SEQ ID NO: 1 or SEQ ID NO: 3 under a hybridized state.

3. A therapeutic agent for cibophobia comprising, as an active ingredient, a substance which shows a specific affinity for a polypeptide consisting of an amino acid sequence shown in SEQ ID NO: 2 or SEQ ID NO: 4 and which inhibits a functional expression of said polypeptide.

4. The therapeutic agent for cibophobia of claim 3, wherein the substance is a nucleic acid.

5. The therapeutic agent for cibophobia of claim 3, wherein the substance is an antibody.

6. A therapeutic agent for cibophobia comprising, as an active ingredient, an expression vector encoding the nucleic acid of claim 2 or 4.

7. A therapeutic agent for cibophobia comprising, as an active ingredient, a host cell transfected with the expression vector of claim 6.

8. A therapeutic agent for a lifestyle-related disease comprising, as an active ingredient, a substance that enhances expression or function of a polypeptide consisting of an amino acid sequence shown in SEQ ID NO: 2 or SEQ ID NO: 4.

9. A therapeutic agent for a lifestyle-related disease comprising, as an active ingredient, a polypeptide of any of the following (a) to (c):
(a) a polypeptide consisting of an amino acid sequence shown in SEQ ID NO: 2 or SEQ ID NO:4
(b) a polypeptide consisting of an amino acid sequence shown in SEQ ID NO: 2 or SEQ ID NO:4, wherein one or more amino acids of the amino acid sequence have been substituted, deleted, inserted, added or modified, which shows a ligand - receptor interaction of the same level as the polypeptide of (a), and which is coupled with a G protein α subunit and shows an activity to promote a GDP/GTP exchange reaction of the subunit
(c) a polypeptide which is an ortholog of the polypeptide of (a).

10. A therapeutic agent for a lifestyle-related disease comprising, as an active ingredient, an expression vector comprising a nucleic acid encoding the polypeptide of claim 9.

11. A therapeutic agent for a lifestyle-related disease comprising, as an active ingredient, a host cell transfected with the expression vector of claim 10.

12. The therapeutic agent for a lifestyle-related disease of any of claims 8 to 11, which is a feeding suppressant, an anti-obesity agent, an anti-diabetic agent or an anti-hyperlipidemic agent.

13. A screening system for a substance having a therapeutic activity against cibophobia or a lifestyle-related disease, which comprises, as one constitution unit, a system comprising, as constituent elements, a lipid bilayer membrane comprising a polypeptide of any of the following (a) to (c):
(a) a polypeptide consisting of an amino acid sequence shown in SEQ ID NO: 2 or SEQ ID NO: 4
(b) a polypeptide consisting of an amino acid sequence shown in SEQ ID NO: 2 or SEQ ID NO: 4, wherein one or more amino acids of the amino acid sequence have been substituted, deleted, inserted, added or modified, which shows a ligand - receptor interaction of the same level as the polypeptide of (a), and which is coupled with a G protein α subunit and shows an activity to promote a GDP/GTP exchange reaction of the subunit,
(c) a polypeptide which is an ortholog of the polypeptide of (a), and a polypeptide comprising at least a receptor-binding region of a G protein α subunit belonging to a certain family and a guanine nucleotide-binding region of any G protein α subunit, wherein said constitution unit is present in a receptor-binding regions of each family of the G protein α subunit.

14. The screening system of claim 13, wherein the constitution unit comprises an eucaryotic host cell transfected with an expression vector comprising a DNA encoding the polypeptide of any of (a) to (c), and an expression vector comprising a DNA encoding a polypeptide comprising at least a receptor-binding region of a G protein α subunit belonging to a certain family and a guanine nucleotide-binding region of any G protein α subunit, a homogenate of said cell or a membrane fraction derived from said cell.

15. The screening system of claim 13, wherein the constitution unit comprises an eucaryotic host cell transfected with an expression vector comprising a DNA encoding a polypeptide fused with a polypeptide comprising, on a C terminal of the polypeptide of any of (a) to (c), at least a receptor-binding region of a G protein α subunit belonging to a certain family and a guanine nucleotide-binding region of any G protein α subunit, a homogenate of said cell or a membrane fraction derived from said cell.

16. The screening system of any of claims 13 to 15, wherein the polypeptide in each constitution unit, which comprises a receptor-binding region of a G protein α subunit and a guanine nucleotide-binding region of any G protein α subunit, further comprises the same effector interacting region and the lipid bilayer membrane further comprises an effector that interacts with said region.

17. The screening system of any of claims 13 to 16, wherein the therapeutic activity against lifestyle-related diseases is a feeding suppressive activity, an anti-obesity activity, an anti-diabetic activity or an anti-hyperlipidemic activity.

18. A screening method for a substance having a therapeutic activity against cibophobia or a lifestyle-related disease, which comprises adding, in each constitution unit of the screening system of any of claims 13 to 15, a labeled GTP analog in the presence and absence of a test substance and comparing an amount of the label bound with a guanine nucleotide-binding region under the both conditions.

19. A screening method for a substance having a therapeutic activity against cibophobia or a lifestyle-related disease, which comprises comparing, in each constitution unit of the screening system of claim 16, an activity of the effector in the presence and absence of the test substance.

20. A method for identifying a G protein α subunit capable of coupling with a polypeptide consisting of an amino acid sequence shown in SEQ ID NO: 2 or SEQ ID NO: 4, which comprises adding, in each constitution unit of the screening system of any of claims 13 to 15, a labeled GTP analog in the presence and absence of a ligand for said polypeptide, and comparing an amount of the label bound with a guanine nucleotide-binding region among constitution units.

21. A method for identifying a G protein α subunit capable of coupling with a polypeptide consisting of an amino acid sequence shown in SEQ ID NO: 2 or SEQ ID NO: 4, which comprises comparing, in each constitution unit of the screening system of claim 16, an activity of the effector in the presence and absence of a ligand for said polypeptide.

22. A screening method for a substance having a therapeutic activity against cibophobia or a lifestyle-related disease, which comprises applying the method of claim 18 or 19 only to a system comprising, as a constituent element, a polypeptide comprising a receptor-binding region of the G protein α subunit as identified by the method of claim 20 or 21.

23. The method of claim 22, wherein the G protein α subunit belongs to a Gs family.

24. A screening system for a ligand for a polypeptide of any of the following (a) to (c):
(a) a polypeptide consisting of an amino acid sequence shown in SEQ ID NO: 2 or SEQ ID NO: 4
(b) a polypeptide consisting of an amino acid sequence shown in SEQ ID NO: 2 or SEQ ID NO: 4, wherein one or more amino acids of the amino acid sequence have been substituted, deleted, inserted, added or modified, which shows a ligand - receptor interaction of the same level as the polypeptide of (a), and which is coupled with a G protein α subunit belonging to a Gs family and shows an activity to promote a GDP/GTP exchange reaction of the subunit,
(c) a polypeptide which is an ortholog of the polypeptide of (a), which comprises, as constituent elements, a lipid bilayer membrane comprising said polypeptide and a polypeptide comprising at least a receptor-binding region of a G protein α subunit belonging to a Gs family and a guanine nucleotide-binding region of any G protein α subunit.

25. The screening system of claim 24, which comprises an eucaryotic host cell transfected with an expression vector comprising a DNA encoding the polypeptide of any of (a) to (c), and an expression vector comprising a DNA encoding a polypeptide comprising at least a receptor-binding region of a G protein α subunit belonging to a Gs family and a guanine nucleotide-binding region of any G protein α subunit, a homogenate of said cell or a membrane fraction derived from said cell.

26. The screening system of claim 24, which comprises an eucaryotic host cell transfected with an expression vector comprising a DNA encoding a polypeptide fused with a polypeptide comprising, on a C terminal side of said polypeptide, any of (a) to (c), at least a receptor-binding region of a G protein α subunit belonging to a Gs family and a guanine nucleotide-binding region of any G protein α subunit, a homogenate of said cell or a membrane fraction derived from said cell.

27. The screening system of any of claims 24 to 26, wherein the polypeptide comprising a receptor-binding region of a G protein α subunit belonging to a Gs family and a guanine nucleotide-binding region of any G protein α subunit further comprises any effector interacting region and the lipid bilayer membrane further comprises an effector that interacts with said region.

28. The screening system of claim 27, wherein the effector is adenylate cyclase.

29. The screening system of any of claims 24 to 28, which is a system for searching a substance having a therapeutic activity against cibophobia or a lifestyle-related disease.

30. The screening system of claim 29, wherein the therapeutic activity against a lifestyle-related disease is a feeding suppressive activity, an anti-obesity activity, an anti-diabetic activity or an anti-hyperlipidemic activity.

31. A screening method for a ligand for a polypeptide of any of the following (a) to (c):
(a) a polypeptide consisting of an amino acid sequence shown in SEQ ID NO: 2 or SEQ ID NO: 4
(b) a polypeptide consisting of an amino acid sequence shown in SEQ ID NO: 2 or SEQ ID NO: 4, wherein one or more amino acids of the amino acid sequence have been substituted, deleted, inserted, added or modified, which shows a ligand - receptor interaction of the same level as the polypeptide of (a), and which is coupled with a G protein α subunit belonging to a Gs family and shows an activity to promote a GDP/GTP exchange reaction of the subunit,
(c) a polypeptide which is an ortholog of the polypeptide of (a),
which comprises adding, in the screening system of any of claims 24 to 26 and in the presence and absence of a test substance, a labeled GTP analog, and comparing the amount of the label bound with a guanine nucleotide-binding region under the both conditions.

32. A screening method for a ligand for a polypeptide of any of the following (a) to (c):
(a) a polypeptide consisting of an amino acid sequence shown in SEQ ID NO: 2 or SEQ ID NO: 4
(b) a polypeptide consisting of an amino acid sequence shown in SEQ ID NO: 2 or SEQ ID NO: 4, wherein one or more amino acids of the amino acid sequence have been substituted, deleted, inserted, added or modified, which shows a ligand - receptor interaction of the same level as the polypeptide of (a), and which is coupled with a G protein α subunit belonging to a Gs family and shows an activity to promote a GDP/GTP exchange reaction of the subunit,
(c) a polypeptide which is an ortholog of the polypeptide of (a),
which comprises comparing an activity of the effector in the screening system of claim 27 in the presence and absence of a test substance.

33. The screening method of claim 32, which comprises comparing an amount of cAMP in an eucaryotic host cell in the presence and absence of a test substance.

34. The screening method of any of claims 31 to 33, which is a system for searching a substance having a therapeutic activity against cibophobia or a lifestyle-related disease.

35. The screening method of any of claims 18, 19, 22, 23 and 34, wherein the therapeutic activity against a lifestyle-related disease is a feeding suppressive activity, an anti-obesity activity, an anti-diabetic activity or an anti-hyperlipidemic activity.

36. A therapeutic agent for cibophobia comprising, as an active ingredient, a substance having a therapeutic activity against cibophobia, which is obtained by the screening method of any of claims 13-16, 18, 19, 22, 23, 29 and 34.

37. A therapeutic agent for a lifestyle-related disease comprising, as an active ingredient, a substance having a therapeutic activity against a lifestyle-related disease, which is obtained by the screening method of any of claims 13-16, 18, 19, 22, 23, 29 and 34.


SEQUENCE LISTING

<110> Sumitomo Pharmaceuticals CO., LTD.

<120> Therapeutic Agent for Anorexia Nervosa or Life-Style Related
Diseases, and Method for Screening Same

<130> K 2082 EP

<150> JP 2001-397523
<151> 2001-12-27

<160> 29

<170> PatentIn version 3.1

<210> 1
<211> 1038
<212> RNA
<213> Homo sapiens

<220>
<221> CDS
<222> (1)..(1035)

<400> 1
aug uac aag gac ugc auc gag ucc acu gga gac uau uuu cuu cuc ugu 48
Met Tyr Lys Asp Cys Ile Glu Ser Thr Gly Asp Tyr Phe Leu Leu Cys
1               5                   10                  15
gac gcc gag ggg cca ugg ggc auc auu cug gag ucc cug gcc aua cuu 96
Asp Ala Glu Gly Pro Trp Gly Ile Ile Leu Glu Ser Leu Ala Ile Leu
            20                  25                  30
ggc auc gug guc aca auu cug cua cuc uua gca uuu cuc uuc cuc aug 144
Gly Ile Val Val Thr Ile Leu Leu Leu Leu Ala Phe Leu Phe Leu Met
        35                  40                  45
cga aag auc caa gac ugc agc cag ugg aau guc cuc ccc acc cag cuc 192
Arg Lys Ile Gln Asp Cys Ser Gln Trp Asn Val Leu Pro Thr Gln Leu
    50                  55                  60
cuc uuc cuc cug agu guc cug ggg cuc uuc gga cuc gcu uuu gcc uuc 240
Leu Phe Leu Leu Ser Val Leu Gly Leu Phe Gly Leu Ala Phe Ala Phe
65                  70                  75                  80
auc auc gag cuc aau caa caa acu gcc ccc gua cgc uac uuu cuc uuu 288
Ile Ile Glu Leu Asn Gln Gln Thr Ala Pro Val Arg Tyr Phe Leu Phe
                85                  90                  95
ggg guu cuc uuu gcu cuc ugu uuc uca ugc cuc uua gcu cau gcc ucc 336
Gly Val Leu Phe Ala Leu Cys Phe Ser Cys Leu Leu Ala His Ala Ser
            100                 105                 110
aau cua gug aag cug guu cgg ggu ugu guc ucc uuc ucc ugg acg aca 384
Asn Leu Val Lys Leu Val Arg Gly Cys Val Ser Phe Ser Trp Thr Thr
        115                 120                 125
auu cug ugc auu gcu auu ggu ugc agu cug uug caa auc auu auu gcc 432
Ile Leu Cys Ile Ala Ile Gly Cys Ser Leu Leu Gln Ile Ile Ile Ala
    130                 135                 140
acu gag uau gug acu cuc auc aug acc aga ggu aug aug uuu gug aau 480
Thr Glu Tyr Val Thr Leu Ile Met Thr Arg Gly Met Met Phe Val Asn
145                 150                 155                 160
aug aca ccc ugc cag cuc aau gug gac uuu guu gua cuc cug guc uau 528
Met Thr Pro Cys Gln Leu Asn Val Asp Phe Val Val Leu Leu Val Tyr
                165                 170                 175
guc cuc uuc cug aug gcc cuc aca uuc uuc guc ucc aaa gcc acc uuc 576
Val Leu Phe Leu Met Ala Leu Thr Phe Phe Val Ser Lys Ala Thr Phe
            180                 185                 190
ugu ggc ccg ugu gag aac ugg aag cag cau gga agg cuc auc uuu auc 624

Cys Gly Pro Cys Glu Asn Trp Lys Gln His Gly Arg Leu Ile Phe Ile
        195                 200                 205
acu gug cuc uuc ucc auc auc auc ugg gug gug ugg auc ucc aug cuc        672
Thr Val Leu Phe Ser Ile Ile Ile Trp Val Val Trp Ile Ser Met Leu
    210                 215                 220
cug aga ggc aac ccg cag uuc cag cga cag ccc cag ugg gac gac ccg        720
Leu Arg Gly Asn Pro Gln Phe Gln Arg Gln Pro Gln Trp Asp Asp Pro
225                 230                 235                 240
guc guc ugc auu gcu cug guc acc aac gca ugg guu uuc cug cug cug        768
Val Val Cys Ile Ala Leu Val Thr Asn Ala Trp Val Phe Leu Leu Leu
                245                 250                 255
uac auc guc ccu gag cuc ugc auu cuc uac aga ucg ugu aga cag gag        816
Tyr Ile Val Pro Glu Leu Cys Ile Leu Tyr Arg Ser Cys Arg Gln Glu
            260                 265                 270
ugc ccu uua caa ggc aau gcc ugc ccc guc aca gcc uac caa cac agc        864
Cys Pro Leu Gln Gly Asn Ala Cys Pro Val Thr Ala Tyr Gln His Ser
        275                 280                 285
uuc caa gug gag aac cag gag cuc ucc aga gcc cga gac agu gau gga        912
Phe Gln Val Glu Asn Gln Glu Leu Ser Arg Ala Arg Asp Ser Asp Gly
    290                 295                 300
gcu gag gag gau gua gca uua acu uca uau ggu acu ccc auu cag ccg        960
Ala Glu Glu Asp Val Ala Leu Thr Ser Tyr Gly Thr Pro Ile Gln Pro
305                 310                 315                 320
cag acu guu gau ccc aca caa gag ugu uuc auc cca cag gcu aaa cua       1008
Gln Thr Val Asp Pro Thr Gln Glu Cys Phe Ile Pro Gln Ala Lys Leu
                325                 330                 335
agc ccc cag caa gau gca gga gga gua uaa                               1038
Ser Pro Gln Gln Asp Ala Gly Gly Val
            340                 345


<210>  2
<211>  345
<212>  PRT
<213>  Homo sapiens

<400>  2
Met Tyr Lys Asp Cys Ile Glu Ser Thr Gly Asp Tyr Phe Leu Leu Cys
1               5                   10                  15

Asp Ala Glu Gly Pro Trp Gly Ile Ile Leu Glu Ser Leu Ala Ile Leu
            20                  25                  30

Gly Ile Val Val Thr Ile Leu Leu Leu Leu Ala Phe Leu Phe Leu Met
        35                  40                  45

Arg Lys Ile Gln Asp Cys Ser Gln Trp Asn Val Leu Pro Thr Gln Leu
    50                  55                  60

Leu Phe Leu Leu Ser Val Leu Gly Leu Phe Gly Leu Ala Phe Ala Phe
65                  70                  75                  80

Ile Ile Glu Leu Asn Gln Gln Thr Ala Pro Val Arg Tyr Phe Leu Phe
                85                  90                  95

Gly Val Leu Phe Ala Leu Cys Phe Ser Cys Leu Leu Ala His Ala Ser
            100                 105                 110

Asn Leu Val Lys Leu Val Arg Gly Cys Val Ser Phe Ser Trp Thr Thr
        115                 120                 125

Ile Leu Cys Ile Ala Ile Gly Cys Ser Leu Leu Gln Ile Ile Ile Ala
    130                 135                 140

Thr Glu Tyr Val Thr Leu Ile Met Thr Arg Gly Met Met Phe Val Asn
145                 150                 155                 160

Met Thr Pro Cys Gln Leu Asn Val Asp Phe Val Val Leu Leu Val Tyr
                165                 170                 175

Val Leu Phe Leu Met Ala Leu Thr Phe Phe Val Ser Lys Ala Thr Phe
            180                 185                 190

Cys Gly Pro Cys Glu Asn Trp Lys Gln His Gly Arg Leu Ile Phe Ile
        195                 200                 205

Thr Val Leu Phe Ser Ile Ile Ile Trp Val Val Trp Ile Ser Met Leu
    210                 215                 220

Leu Arg Gly Asn Pro Gln Phe Gln Arg Gln Pro Gln Trp Asp Asp Pro
225                 230                 235                 240

Val Val Cys Ile Ala Leu Val Thr Asn Ala Trp Val Phe Leu Leu Leu
                245                 250                 255

Tyr Ile Val Pro Glu Leu Cys Ile Leu Tyr Arg Ser Cys Arg Gln Glu
            260                 265                 270

Cys Pro Leu Gln Gly Asn Ala Cys Pro Val Thr Ala Tyr Gln His Ser
        275                 280                 285

Phe Gln Val Glu Asn Gln Glu Leu Ser Arg Ala Arg Asp Ser Asp Gly
    290                 295                 300

Ala Glu Glu Asp Val Ala Leu Thr Ser Tyr Gly Thr Pro Ile Gln Pro
305                 310                 315                 320

Gln Thr Val Asp Pro Thr Gln Glu Cys Phe Ile Pro Gln Ala Lys Leu
                325                 330                 335

Ser Pro Gln Gln Asp Ala Gly Gly Val
            340                 345


<210>  3
<211>  1324
<212>  RNA
<213>  Mus musculus

<220>
<221>  CDS
<222>  (148)..(1047)

<400>  3
gaggagagau uaaagcaccu gaaguugucu ccuccuuacu cauuugcagc cacagcuucc      60

ccaccagcac agccucagag gcuuccggag uagacucgga ggaggagacc agacauccgu     120

ucucgugagg ucuaccuaua agucacc aug uau gag gac ugc gug aag ucc aca     174
                              Met Tyr Glu Asp Cys Val Lys Ser Thr
                              1               5

gaa gac uau uac cuc uuc ugu gac aac gag ggg cca ugg gcc auu guu       222
Glu Asp Tyr Tyr Leu Phe Cys Asp Asn Glu Gly Pro Trp Ala Ile Val
10                  15                  20                  25

cug gag ucc uug gca gug auu ggc aua gug guu acc aua uug cua cuc       270
Leu Glu Ser Leu Ala Val Ile Gly Ile Val Val Thr Ile Leu Leu Leu
                30                  35                  40

cug gca uuu cug uuc cuc aug cgg aag guu cag gac ugc agc cag ugg       318

Leu Ala Phe Leu Phe Leu Met Arg Lys Val Gln Asp Cys Ser Gln Trp
            45                  50                  55
aac gug cuu ccc acu cag uuc cuc uuc cug cug gcu gug cuc ggg cuc 366
Asn Val Leu Pro Thr Gln Phe Leu Phe Leu Leu Ala Val Leu Gly Leu
        60                  65                  70
uuc gga cuu acu uuu gcc uuc auc auc caa cuc aac cau caa acu gcc 414
Phe Gly Leu Thr Phe Ala Phe Ile Ile Gln Leu Asn His Gln Thr Ala
    75                  80                  85
ccu guu cgc uac uuc cuc uuu ggg guu cuc uuu gcu auc ugc uuc ucc 462
Pro Val Arg Tyr Phe Leu Phe Gly Val Leu Phe Ala Ile Cys Phe Ser
90                  95                  100                 105
ugc cuc cug gcu cau gcc ucc aac cug gug aag cug guc cgg ggu aga 510
Cys Leu Leu Ala His Ala Ser Asn Leu Val Lys Leu Val Arg Gly Arg
                110                 115                 120
guc ucc uuc ugc ugg aca aca auu cug uuc auu gcu auc ggu guc agc 558
Val Ser Phe Cys Trp Thr Thr Ile Leu Phe Ile Ala Ile Gly Val Ser
            125                 130                 135
cug uug cag acc auc auu gcg aua gag uau gug acc cuc auc aug acc 606
Leu Leu Gln Thr Ile Ile Ala Ile Glu Tyr Val Thr Leu Ile Met Thr
        140                 145                 150
aga ggc uug aug uuc gag cau aug aca ccg uau cag cuc aau gug gac 654
Arg Gly Leu Met Phe Glu His Met Thr Pro Tyr Gln Leu Asn Val Asp
    155                 160                 165
uuu guc ugu cuc cug auc uau guc cuc uuc cug aug gcc cuc acu uuc 702
Phe Val Cys Leu Leu Ile Tyr Val Leu Phe Leu Met Ala Leu Thr Phe
170                 175                 180                 185
uuc guc ucc aag gcc acc uuc ugu ggc cca ugu gag aac ugg aaa cag 750
Phe Val Ser Lys Ala Thr Phe Cys Gly Pro Cys Glu Asn Trp Lys Gln
                190                 195                 200
cac gga agg cuc aua uuu gcu acu gug cug guc ucu auc auu auc ugg 798
His Gly Arg Leu Ile Phe Ala Thr Val Leu Val Ser Ile Ile Ile Trp
            205                 210                 215
gug gug ugg auc ucc aug cuc uug aga ggc aac ccc cag cuc cag cga 846
Val Val Trp Ile Ser Met Leu Leu Arg Gly Asn Pro Gln Leu Gln Arg
        220                 225                 230
cag ccc cac ugg gac gau gca guc auc ugc auu ggc cug guc acc aac 894
Gln Pro His Trp Asp Asp Ala Val Ile Cys Ile Gly Leu Val Thr Asn
    235                 240                 245
gcu ugg guc uuc cug cug auc uac auc auc ccu gag cug agc aua cuc 942
Ala Trp Val Phe Leu Leu Ile Tyr Ile Ile Pro Glu Leu Ser Ile Leu
250                 255                 260                 265
uac agg uca ugu agg cag gag ugu ccu aca caa ggc aac guc ugc cag 990
Tyr Arg Ser Cys Arg Gln Glu Cys Pro Thr Gln Gly Asn Val Cys Gln
                270                 275                 280
guc ccu guc uac caa cgc agc uuc agg aug gau acc cag gaa ccc acc 1038
Val Pro Val Tyr Gln Arg Ser Phe Arg Met Asp Thr Gln Glu Pro Thr
            285                 290                 295
aga gag ugc ugaucccagc cgggaguauc ucaucccauc agcuacacua 1087
Arg Glu Cys
        300
agcccacagc aagaugcagg auuguaaagc uacuggaaac agcauagaga caaccuggaa 1147
gagugcccug cuccacacag cccuaaagag cccaggggag cacuggacac acugucaaug 1207
aagcauccuu ccugucccuu ccucucuguu ucccugcccu uuccacucuu cuggacccag 1267
ccucugaaga cugucauguc cugcacaauu aaaaucuugu ugccacccua aaaaaaa 1324

<210>  4
<211>  300
<212>  PRT
<213>  Mus musculus

<400>  4
Met Tyr Glu Asp Cys Val Lys Ser Thr Glu Asp Tyr Tyr Leu Phe Cys
1               5                   10                  15

Asp Asn Glu Gly Pro Trp Ala Ile Val Leu Glu Ser Leu Ala Val Ile
            20                  25                  30

Gly Ile Val Val Thr Ile Leu Leu Leu Leu Ala Phe Leu Phe Leu Met
        35                  40                  45

Arg Lys Val Gln Asp Cys Ser Gln Trp Asn Val Leu Pro Thr Gln Phe
    50                  55                  60

Leu Phe Leu Leu Ala Val Leu Gly Leu Phe Gly Leu Thr Phe Ala Phe
65                  70                  75                  80

Ile Ile Gln Leu Asn His Gln Thr Ala Pro Val Arg Tyr Phe Leu Phe
                85                  90                  95

Gly Val Leu Phe Ala Ile Cys Phe Ser Cys Leu Leu Ala His Ala Ser
            100                 105                 110

Asn Leu Val Lys Leu Val Arg Gly Arg Val Ser Phe Cys Trp Thr Thr
        115                 120                 125

Ile Leu Phe Ile Ala Ile Gly Val Ser Leu Leu Gln Thr Ile Ile Ala
    130                 135                 140

Ile Glu Tyr Val Thr Leu Ile Met Thr Arg Gly Leu Met Phe Glu His
145                 150                 155                 160

Met Thr Pro Tyr Gln Leu Asn Val Asp Phe Val Cys Leu Leu Ile Tyr
                165                 170                 175

Val Leu Phe Leu Met Ala Leu Thr Phe Phe Val Ser Lys Ala Thr Phe
            180                 185                 190

Cys Gly Pro Cys Glu Asn Trp Lys Gln His Gly Arg Leu Ile Phe Ala
        195                 200                 205

Thr Val Leu Val Ser Ile Ile Ile Trp Val Val Trp Ile Ser Met Leu
    210                 215                 220

Leu Arg Gly Asn Pro Gln Leu Gln Arg Gln Pro His Trp Asp Asp Ala
225                 230                 235                 240

Val Ile Cys Ile Gly Leu Val Thr Asn Ala Trp Val Phe Leu Leu Ile
                245                 250                 255

Tyr Ile Ile Pro Glu Leu Ser Ile Leu Tyr Arg Ser Cys Arg Gln Glu
            260                 265                 270

Cys Pro Thr Gln Gly Asn Val Cys Gln Val Pro Val Tyr Gln Arg Ser
        275                 280                 285

Phe Arg Met Asp Thr Gln Glu Pro Thr Arg Glu Cys
    290                 295                 300

<210> 5
<211> 20
<212> DNA
<213> Artificial/Unknown

<220>
<221> misc_feature
<223> Oligonucleotide designed to act as primer for amplifying GPRC5D
      mRNA.

<400> 5
acctcttctg tgacaacgag                                                    20


<210> 6
<211> 20
<212> DNA
<213> Artificial/Unknown

<220>
<221> misc_feature
<223> Oligonucleotide designed to act as primer for amplifying GPRC5D
      mRNA.

<400> 6
ggaagaggac atagatcagg                                                    20


<210> 7
<211> 25
<212> DNA
<213> Artificial/Unknown

<220>
<221> misc_feature
<223> Oligonucleotide designed to act as antisense DNA for inhibiting
      expression of GPRC5D.

<400> 7
tcatacatgg tgacttatag gtaga                                              25


<210> 8
<211> 25
<212> DNA
<213> Artificial/Unknown

<220>
<221> misc_feature
<223> Oligonucleotide designed to act as antisense DNA for sequence
      resulted from mutation causing abnormal splicing at position 705
      of beta-globin pre-mRNA in thalassemia.

<400> 8
cctcttacct cagttacaat ttata                                              25


<210> 9
<211> 25
<212> DNA
<213> Artificial/Unknown

<220>
<221> misc_feature

<223>  Oligonucleotide designed to act as primer for amplifying GPRC5D
      mRNA.

<400>  9
ggagtatctc atcccatcag ctaca                                          25


<210>  10
<211>  24
<212>  DNA
<213>  Artificial/Unknown

<220>
<221>  misc_feature
<223>  Oligonucleotide designed to act as primer for amplifying GPRC5D
      mRNA.

<400>  10
cactcttcca ggttgtctct atgc                                           24


<210>  11
<211>  22
<212>  DNA
<213>  Artificial/Unknown

<220>
<221>  misc_feature
<223>  Oligonucleotide designed to act as primer for amplifying GAPDH
      mRNA.

<400>  11
caagagaggc cctatcccaa ct                                             22


<210>  12
<211>  23
<212>  DNA
<213>  Artificial/Unknown

<220>
<221>  misc_feature
<223>  Oligonucleotide designed to act as primer for amlifying GAPDH
      mRNA.

<400>  12
ctaggcccct cctgttatta tgg                                            23


<210>  13
<211>  20
<212>  DNA
<213>  Artificial

<220>
<221>  misc_feature
<223>  Oligonucleotide designed to act as sense primer for amplifying
      human G protein Ga16 cDNA fragment containing full length ORF.

<400>  13
ccatggcccg ctcgctgacc '                                              20


<210>  14

<211>  21
<212>  DNA
<213>  Artificial

<220>
<221>  misc_feature
<223>  Oligonucleotide designed to act as antisense primer for
       amplifying human G protein Ga16 cDNA fragment containing full
       length ORF.

<400>  14
ccgaggctgg agagatagac c                                              21


<210>  15
<211>  19
<212>  DNA
<213>  Artificial

<220>
<221>  misc_feature
<223>  Oligonucleotide designed to act as sense primer for amplifying
       human G protein Gai2 cDNA fragment containing full length ORF.

<400>  15
gcggcggagc ggcggaacg                                                 19


<210>  16
<211>  24
<212>  DNA
<213>  Artificial

<220>
<221>  misc_feature
<223>  Oligonucleotide designed to act as antisense primer for
       amplifying human G protein Gai2 cDNA fragment containing full
       length ORF.

<400>  16
ggagaaaagc ggcgggggaa cagg                                           24


<210>  17
<211>  21
<212>  DNA
<213>  Artificial

<220>
<221>  misc_feature
<223>  Oligonucleotide designed to act as sense primer for amplifying
       human G protein GaS2 cDNA fragment containing full length ORF.

<400>  17
ccatgggctg cctcgggaac a                                              21


<210>  18
<211>  23
<212>  DNA
<213>  Artificial

<220>
<221>  misc_feature

<223>  Oligonucleotide designed to act as antisense primer for
      amplifying human G protein GaS2 cDNA fragment containng full
      length ORF.

<400>  18
ggtttcgcaa aatcactcgg ggg                                              23


<210>  19
<211>  21
<212>  DNA
<213>  Artificial

<220>
<221>  misc_feature
<223>  Oligonucleotide designed to act as sense primer for amplifying
       human G protein Ga16 cDNA fragment from initiation codon.

<400>  19
atggcccgct cgctgacctg g                                                21


<210>  20
<211>  21
<212>  DNA
<213>  Artificial

<220>
<221>  misc_feature
<223>  Oligonucleotide designed to act as sense primer for amplifying
       human G protein Gai2 cDNA fragment from initiation codon.

<400>  20
atgggctgca ccgtgagcgc c                                                21


<210>  21
<211>  20
<212>  DNA
<213>  Artificial

<220>
<221>  misc_feature
<223>  Oligonucleotide designed to act as sense primer for amplifying
       human G protein GaS2 cDNA fragment from initiation codon.

<400>  21
atgggctgcc tcgggaacag                                                  20


<210>  22
<211>  18
<212>  DNA
<213>  Artificial

<220>
<221>  misc_feature
<223>  Oligonucleotide designed to act as antisense primer for
       amplifying multiple cloning site of plasmid pcDNA3.1(+).

<400>  22
tagaaggcac agtcgagg                                                    18

<210>  23
<211>  24
<212>  DNA
<213>  Artificial

<220>
<221>  misc_feature
<223>  Sense strand oligonucleotide designed to construct linker
       containing nucleotide sequence encoding 6xHis-tag peptide
       sequence.

<400>  23
gatatccatc atcatcatca ccat                                              24


<210>  24
<211>  18
<212>  DNA
<213>  Artificial

<220>
<221>  misc_feature
<223>  Antisense strand oligonucleotide desined to construct linker
       containing nucleotide sequence encoding 6xHis-tag peptide
       sequence.

<400>  24
atggtgatga tgatgatg                                                     18


<210>  25
<211>  20
<212>  DNA
<213>  Artificial

<220>
<221>  misc_feature
<223>  Oligonucleotide designed to act as sence primer for amplifying
       GPRC5D cDNA.

<400>  25
ggagaagggc atcagaaaac                                                   20


<210>  26
<211>  22
<212>  DNA
<213>  Artificial

<220>
<221>  misc_feature
<223>  Oligonucleotide designed to act as antisence primer for
       amplifying GPRC5D cDNA.

<400>  26
ttatactcct cctgcatctt gc                                                22


<210>  27
<211>  58
<212>  DNA
<213>  Artificial

<220>

<221>  misc_feature
<223>  Oligonucleotide designed to act as sence primer for
      amplifying ORF of GPRC5D cDNA.

<400>  27
ggggacaagt ttgtacaaaa aagcaggctc caccatgtac aaggactgca tcgagtcc        58


<210>  28
<211>  55
<212>  DNA
<213>  Artificial

<220>
<221>  misc_feature
<223>  Oligonucleotide designed to act as antisense primer for
      amplifying ORF (R form) of GPRC5D cDNA.

<400>  28
ggggaccact ttgtacaaga aagctgggtc attatactcc tcctgcatct tgctg        55


<210>  29
<211>  51
<212>  DNA
<213>  Artificial

<220>
<221>  misc_feature
<223>  Oligonucleotide designed to act as antisense primer for
      amplifying ORF (F form) of GPRC5D cDNA.

<400>  29
ggggaccact ttgtacaaga aagctgggtc tactcctcct gcatcttgct g        51